# EUROPEAN PATENT SPECIFICATION

(11) **EP 4 330 971 B1**
(45) Date of publication and mention of the grant of the patent: **18.02.2026**
(21) Application number: 23809951.9
(22) Date of filing: 25.06.2023
(51) Int. Cl.: G16H 15/00, G06T 7/00, G06T 7/33

(54) **SYSTEMS AND METHODS FOR IMAGE PROCESSING**
SYSTEME UND VERFAHREN ZUR BILDVERARBEITUNG
SYSTÈMES ET PROCÉDÉS DE TRAITEMENT D'IMAGE

(30) Priority: 24.06.2022 CN 202210731216; 30.06.2022 CN 202210763718; 24.06.2022 CN 202210722850
(43) Date of publication of application: 06.03.2024
(73) Proprietor: Shanghai United Imaging Healthcare Co., Ltd., Shanghai 201807 (CN)
(72) Inventor: LIU, Shitao, Shanghai 201807 (CN); XU, Tianyi, Shanghai 201807 (CN); LI, Jinlong, Shanghai 201807 (CN)
(74) Representative: Wang, Bo
(86) International application number: PCT/CN2023/102157
(87) International publication number: WO 2023/246937

(56) References cited:
- WO-A1-2011/010231
- CN-A- 104 584 072
- CN-A- 109 543 059
- CN-A- 113 130 050
- CN-A- 113 539 439
- CN-A- 114 119 491
- CN-A- 114 913 191
- CN-A- 115 187 685
- CN-A- 115 762 696
- US-A1- 2017 243 349
- AUVITY SYLVAIN ET AL: "Repurposing radiotracers for myelin imaging: a study comparing 18F-florbetaben, 18F-florbetapir, 18F-flutemetamol,11C-MeDAS, and 11C-PiB", EUROPEAN JOURNAL OF NUCLEAR MEDICINE AND MOLECULAR IMAGING, SPRINGER BERLIN HEIDELBERG, BERLIN/HEIDELBERG, vol. 47, no. 2, 4 November 2019 (2019-11-04), pages 490 - 501, XP036993334, ISSN: 1619-7070, [retrieved on 20191104], DOI: 10.1007/S00259-019-04516-Z
- WEIWEI RUAN ET AL: "Regional SUV quantification in hybrid PET/MR, a comparison of two atlas-based automatic brain segmentation methods", EJNMMI RESEARCH, BIOMED CENTRAL LTD, LONDON, UK, vol. 10, no. 1, 8 June 2020 (2020-06-08), pages 1 - 9, XP021277713, DOI: 10.1186/S13550-020-00648-8
- TOMOHIRO KANETA ET AL: "A modified method of 3D-SSP analysis for amyloid PET imaging using [11C]BF-227", ANNALS OF NUCLEAR MEDICINE, vol. 25, no. 10, 27 July 2011 (2011-07-27), pages 732 - 739, XP055050461, ISSN: 0914-7187, DOI: 10.1007/s12149-011-0518-7

## Description

### TECHNICAL FIELD

The present disclosure generally relates to image processing, and more particularly, relates to systems and methods for image analysis and/or image display.

### BACKGROUND

Medical imaging becomes more and more important in modern medicine. Imaging processing, such as imaging analysis and/or image display, often needs to be performed on medical images to help doctors to make diagnoses. Therefore, it is desirable to provide systems and methods to realize more accurate and more efficient image analysis and/or image display. Relevant prior art is disclosed in AUVITY SYLVAIN ET AL: "Repurposing radiotracers for myelin imaging: a study comparing 18F-florbetaben, 18F-florbetapir, 18F-flutemetamol, 11 C-MeDAS, and 11 C-PiB", EUROPEAN JOURNAL OF NUCLEAR MEDICINE AND MOLECULAR IMAGING, SPRINGER BERLIN HEIDELBERG, BERLIN/HEIDELBERG, vol. 47, no. 2, 4 November 2019 (2019-11-04), pages 490-501, ISSN: 1619-7070, DOI: 10.1007/ S00259-019-04516-Z, which is to provide a head-to-head comparison of 18F-florbetaben, 18F-florbetapir, 18F-flutemetamol, 11C-MeDAS, and 11C-PiB with regard to brain kinetics and binding in white matter (WM). Relevant prior art is disclosed in US 2017/243349 A1 which relates to a method for dynamic contrast enhanced (DCE) image processing and kinetic modeling of an organ's region-of-interest.

### SUMMARY

The invention is set out in the appended set of claims.

### BRIEF DESCRIPTION OF THE DRAWINGS

The present disclosure is further described in terms of exemplary embodiments. These exemplary embodiments are described in detail with reference to the drawings. The drawings are not to scale. These embodiments are non-limiting exemplary embodiments, in which like reference numerals represent similar structures throughout the several views of the drawings, and wherein:
FIG. 1 is a schematic diagram illustrating an exemplary medical system according to some embodiments of the present disclosure;
FIG. 2 is a block diagram illustrating an exemplary processing device according to some embodiments of the present disclosure;
FIG. 3 is a flowchart illustrating an exemplary image analysis process according to some embodiments of the present disclosure;
FIG. 4 is a schematic diagram illustrating an exemplary image analysis process according to some embodiments of the present disclosure;
FIG. 5 is a schematic diagram illustrating an exemplary process for determining a standard space and a standard uptake variation curve according to some embodiments of the present disclosure;
FIG. 6 is a flowchart illustrating an exemplary image display process according to some embodiments of the present disclosure;
FIG. 7 is a schematic diagram illustrating an exemplary process for determining subranges and second value ranges according to some embodiments of the present disclosure;
FIG. 8 is a schematic diagram illustrating an exemplary target image according to some embodiments of the present disclosure;
FIG. 9 is a schematic diagram illustrating an exemplary image display process according to some embodiments of the present disclosure;
FIG. 10 is a schematic diagram illustrating an exemplary process for generating a second target image according to some embodiments of the present disclosure;
FIG. 11 is a schematic diagram illustrating an exemplary second target image according to some embodiments of the present disclosure; and
FIG. 12 is a flowchart illustrating an exemplary image processing process according to some embodiments of the present disclosure.

### DETAILED DESCRIPTION

In the following detailed description, numerous specific details may be set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high-level, without detail, in order to avoid unnecessarily obscuring aspects of the present disclosure. The general principles defined herein may be applied to other embodiments and applications without departing from the scope of the present disclosure. Thus, the present disclosure may be not limited to the embodiments shown, but to be accorded the widest scope consistent with the claims.

The terminology used herein may be for the purpose of describing particular example embodiments only and may be not intended to be limiting. As used herein, the singular forms "a," "an," and "the" may be intended to include the plural forms as well, unless the context clearly indicates otherwise. It may be understood that the terms "system," "unit," "module," and/or "block" used herein are one method to distinguish different components, elements, parts, sections or assemblies of different levels in ascending order. However, the terms may be displaced by another expression if they achieve the same purpose.

The modules (or units, blocks, units) described in the present disclosure may be implemented as software and/or hardware modules and may be stored in any type of non-transitory computer-readable medium or other storage devices. In some embodiments, a software module may be compiled and linked into an executable program. It may be appreciated that software modules may be callable from other modules or from themselves, and/or may be invoked in response to detected events or interrupts. Software modules configured for execution on computing devices may be provided on a computer readable medium or as a digital download (and can be originally stored in a compressed or installable format that requires installation, decompression, or decryption prior to execution). Such software code may be stored, partially or fully, on a memory device of the executing computing device, for execution by the computing device. Software instructions may be embedded in a firmware, such as an EPROM. It may be further appreciated that hardware modules (e.g., circuits) may be included in connected or coupled logic units, such as gates and flip-flops, and/or may be included in programmable units, such as programmable gate arrays or processors. The modules or computing device functionality described herein may be preferably implemented as hardware modules, but may be software modules as well. In general, the modules described herein refer to logical modules that may be combined with other modules or divided into units despite their physical organization or storage.

Certain terminology has been used to describe embodiments of the present disclosure. For example, the terms "one embodiment," "an embodiment," and/or "some embodiments" may mean that a particular feature, structure or characteristic described in connection with the embodiment is in at least one embodiment of the present disclosure. Therefore, it is emphasized and should be appreciated that two or more references to "an embodiment" or "one embodiment" or "an alternative embodiment" in various portions of this specification may not necessarily all referring to the same embodiment. Furthermore, the particular features, structures or characteristics may be combined as suitable in one or more embodiments of the present disclosure.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings may be for the purpose of illustration and description only and may be not intended to limit the scope of the present disclosure.

The flowcharts used in the present disclosure may illustrate operations that systems implement according to some embodiments of the present disclosure. It is to be expressly understood, the operations of the flowcharts may be implemented not in order. Conversely, the operations may be implemented in inverted order, or simultaneously. Moreover, one or more other operations may be added to the flowcharts. One or more operations may be removed from the flowcharts. In any case, the possible modifications to the flowcharts have to be within the scope of the appended claims.

In order to obtain blood flow and metabolism, a dynamic positron emission tomography (PET) scan is performed on a target subject (e.g., a patient) that is injected with a tracer to obtain multiple PET images. Further, a user (e.g., a medical staff) needs to mark the contour of a region of interest (ROI) (e.g., a liver region) in each of the multiple PET images, the tracer distribution information may be extracted based on the ROI. The user may evaluate the blood flow and metabolism by analyzing tracer distribution information in ROIs of the multiple PET images. The above method relies on artificial experience, and the ROIs marked by different people or the same person on different images are often inconsistent, thereby resulting in low analysis efficiency and inaccurate analysis results.

The present disclosure provides systems and methods for image analysis. The systems may obtain a plurality of images of a target subject that are captured consecutively over time. The target subject may be injected with a tracer and include a target body portion (e.g., an ROI). For each of the plurality of images, the systems may generate a sub-image of the target body portion by segmenting the image and transform the sub-image to generate a transformed sub-image in a standard space based on a template image of the target body portion. The standard space may include a plurality of template images of a plurality of body portions of the target subject arranged in a preset order. The systems may determine an uptake variation curve indicating a change of tracer uptake in the target body portion over the time based on the transformed sub-images corresponding to the plurality of images. Further, the systems may obtain a standard uptake variation curve corresponding to the target body portion and generate a comparison result between the uptake variation curve and the standard uptake variation curve. Based on the comparison result, the blood flow and metabolism of the target subject may be evaluated.

According to the embodiments of the present disclosure, the sub-images of the target body portion may be transformed into the standard space, which may reduce the impact of differences in the morphology of the target body portions of different target subjects on the analysis of blood flow and metabolism, thereby improving the evaluation accurate of the blood flow and metabolism.

A PET image may be displayed based on a value range of grayscale or color scale that corresponds to a value range of tracer standard uptake value (SUV). Traditionally, all values in the SUV range are transformed to values in a preset grayscale value range (e.g., 0-255) or a color scale range by performing, for example, a linear transformation or an on-linear transformation. However, when there are one or more physical points that have high SUV values(e.g., 100), the pixels or voxels corresponding to these physical points and the pixels or voxels corresponding to the other physical points may have very different gray values or color values. In such cases, the PET image may easily include image distortion problems, for example, a decrease in contrast and drastic changes in pixel values in some ROIs, thereby reducing the clarity and readability of the displayed PET image.

The present disclosure provides systems and methods for image display. The systems may divide a first value range of a first parameter into multiple subranges. The subranges may include at least one target subrange and a non-target subrange. The first parameter may relate to tracer uptake (e.g., a tracer uptake concentration or the SUV) of a plurality of physical points of a target subject in a medical scan of the target subject. For each of the subranges, the systems may determine a second value range of a second parameter corresponding to the subrange. The second parameter may relate to an image feature (e.g., the grayscale or color scale) of the plurality of physical points in a target image of the target subject acquired by the medical scan. A span of the second value range of each of the at least one target subrange may be greater than a span of the second value range of the non-target subrange. Further, the systems may generate the target image of the target subject based on scan data acquired in the medical scan and the second value ranges of the second parameter corresponding to each of the subranges.

According to the embodiments of the present disclosure, the second value range of the second parameter corresponding to each subrange (e.g., the at least one target subrange, the non-target subrange) may be determined. For example, the high SUV values may be in the non-target subrange, and the span of the second value range of each of the at least one target subrange may be greater than the span of the second value range of the non-target subrange, and therefore the high SUV values do not affect the display of the PET image, thereby improving the clarity and readability of the displayed PET image.

At present, the connection of various workstations has been realized, and medical images can be obtained and patient information can be retrieved from other servers based on a user instruction, and patient information does not affect the display of the medical images. Therefore, there are problems such as high labor input cost, low processing efficiency, and easy operation errors, and the medical image cannot fully reflect the patient's condition, resulting in that doctors are unable to make effective diagnoses.

The present disclosure provides systems and methods for image display. The systems may obtain basic information of a target subject and disease risk information of the target subject based on the basic information of the target subject from a disease information database. The disease information database may store basic information and disease risk information of a plurality of subjects. The disease risk information of each of the plurality of subjects may indicate whether the subject has a high risk of disease. Further, the systems may generate a second target image of the target subject based on the disease risk information of the target subject. For example, the systems may determine one or more disease risk portions of the target subject based on the disease risk information and display the one or more disease risk portions in the second target image using a highlight mask or an annotation.

According to the embodiments of the present disclosure, the second target image is automatically displayed based on the obtained disease risk information of the target subject, which does not require human intervention and improves the efficiency, the rationality, and the quality of the display of the second target image.

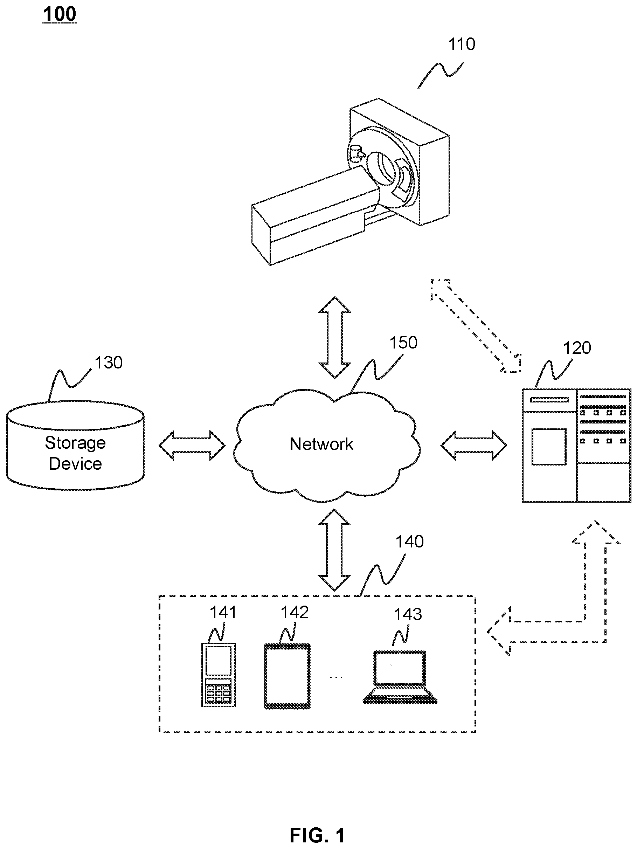

FIG. 1 is a schematic diagram illustrating an exemplary medical system 100 according to some embodiments of the present disclosure. As shown in FIG. 1, the medical system 100 may include an imaging device 110, a processing device 120, a storage device 130, one or more terminals 140, and a network 150. In some embodiments, the imaging device 110, the processing device 120, the storage device 130, and/or the terminal(s) 140 may be connected to and/or communicate with each other via a wireless connection, a wired connection, or a combination thereof.

The imaging device 110 may be configured to scan a target subject (or a part of the subject) to acquire medical image data associated with the target subject. The medial image data relating to the target subject may be used for generating a medical image (e.g., a PET image) of the target subject. The medical image may illustrate an internal structure and the health condition of the target subject. In some embodiments, the imaging device 110 may include a single-modality scanner and/or multi-modality scanner. The single modality scanner may include, for example, a positron emission tomography (PET) scanner, an X-ray scanner, a CT scanner, a magnetic resonance imaging (MRI) scanner, an ultrasonography scanner, a Digital Radiography (DR) scanner, or the like, or any combination thereof. The multi-modality scanner may include, for example, a positron emission tomography-X-ray imaging (PET-X-ray) scanner, a positron emission tomography-computed tomography (PET-CT) scanner, a single-photon emission computed tomography-magnetic resonance imaging (SPECT-MRI) scanner, an X-ray imaging-magnetic resonance imaging (X-ray-MRI) scanner, etc. It should be noted that the imaging device 110 described below is merely provided for illustration purposes, and not intended to limit the scope of the present disclosure.

In some embodiments, the processing device 120 may be a single server or a server group. The server group may be centralized or distributed. The processing device 120 may process data and/or information obtained from the imaging device 110, the storage device 130, and/or the terminal(s) 140. For example, the processing device 120 may determine an uptake variation curve indicating a change of tracer uptake in a target body portion of a target subject over time. As another example, the processing device 120 may generate a target image (also referred to as a first target image) of the target subject based on scan data acquired in a medical scan of the target subject and second value ranges of a second parameter relating to image feature of the plurality of physical points in the target image. As yet another example, the processing device 120 may obtain disease risk information of the target subject and generate a second target image of the target subject based on the disease risk information of the target subject.

In some embodiments, the processing device 120 may be local or remote from the medical system 100. In some embodiments, the processing device 120 may be implemented on a cloud platform. In some embodiments, the processing device 120 or a portion of the processing device 120 may be integrated into the imaging device 110 and/or the terminal(s) 140. It should be noted that the processing device 120 in the present disclosure may include one or multiple processors. Thus operations and/or method steps that are performed by one processor may also be jointly or separately performed by the multiple processors.

The storage device 130 may store data, instructions, and/or any other information. In some embodiments, the storage device 130 may store data obtained from the imaging device 110, the processing device 120, and/or the terminal(s) 140. In some embodiments, the storage device 130 may store data and/or instructions that the processing device 120 may execute or use to perform exemplary methods described in the present disclosure. In some embodiments, the storage device 130 may include a mass storage device, a removable storage device, a volatile read-and-write memory, a read-only memory (ROM), or the like, or a combination thereof. In some embodiments, the storage device 130 may be implemented on a cloud platform. In some embodiments, the storage device 130 may be part of the imaging device 110, the processing device 120, and/or the terminal(s) 140.

The terminal(s) 140 may be configured to enable a user interaction between a user and the medical system 100. In some embodiments, the terminal(s) 140 may be connected to and/or communicate with the imaging device 110, the processing device 120, and/or the storage device 130. In some embodiments, the terminal(s) 140 may include a mobile device 141, a tablet computer 142, a laptop computer 143, or the like, or a combination thereof. In some embodiments, the terminal(s) 140 may be part of the processing device 120 and/or the imaging device 110.

The network 150 may include any suitable network that can facilitate the exchange of information and/or data for the medical system 100. In some embodiments, one or more components of the medical system 100 (e.g., the imaging device 110, the processing device 120, the storage device 130, the terminal(s) 140, etc.) may communicate information and/or data with one or more other components of the medical system 100 via the network 150.

It should be noted that the above description is intended to be illustrative, and not to limit the scope of the present disclosure. Many alternatives and variations will be apparent to those skilled in the art. The features, structures, methods, and characteristics of the exemplary embodiments described herein may be combined in various ways to obtain additional and/or alternative exemplary embodiments. In any case, all such modifications and alternatives are possible within the scope of the appended claims. In some embodiments, the medical system 100 may include one or more additional components and/or one or more components described above may be omitted. Additionally or alternatively, two or more components of the medical system 100 may be integrated into a single component. For example, the processing device 120 may be integrated into the imaging device 110. As another example, a component of the medical system 100 may be replaced by another component that can implement the functions of the component. However, those variations do not depart from the scope of the present disclosure.

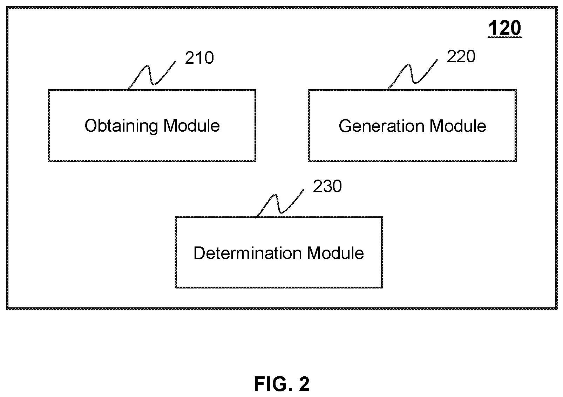

FIG. 2 is a block diagram illustrating an exemplary processing device according to some embodiments of the present disclosure. As shown in FIG. 2, the processing device 120 may include an obtaining module 210, a generation module 220, and a determination module 230.

The processing device is configured to perform an image analysis process. Specifically, the obtaining module 210 is configured to obtain a plurality of images of a target subject that are captured consecutively over time. The generation module 220 is configured to generate a sub-image of the target body portion by segmenting the image. The determination module 230 is configured to transform the sub-image of the target body portion to generate a transformed sub-image of the target body portion in a standard space based on a template image of the target body portion. The determination module 230 is further configured to determine an uptake variation curve indicating a change of tracer uptake in the target body portion over the time based on the transformed sub-images corresponding to the plurality of images. The obtaining module 210 is further configured to obtain a standard uptake variation curve corresponding to the target body portion. The generation module 220 may be further configured to generate a comparison result between the uptake variation curve and the standard uptake variation curve. More descriptions regarding the image analysis process may be found elsewhere in the present disclosure (e.g., operations 310-360 and the description thereof).

In some embodiments, the processing device 120 may be configured to perform an image display process. Specifically, the determination module 230 may be further configured to divide a first value range of a first parameter into multiple subranges and determine a second value range of a second parameter corresponding to the subrange. The generation module 220 may be further configured to generate a target image of the target subject based on scan data acquired in the medical scan and the second value range of the second parameter corresponding to each of the subranges. More descriptions regarding the image analysis process may be found elsewhere in the present disclosure (e.g., operations 610-630 and the description thereof).

In some embodiments, the processing device 120 may be configured to perform an image display process. Specifically, the obtaining module 210 may be further configured to obtain basic information of a target subject and disease risk information of the target subject based on the basic information of the target subject from a disease information database. The generation module 220 may be further configured to generate a second target image of the target subject based on the disease risk information of the target subject. More descriptions regarding the image analysis process may be found elsewhere in the present disclosure (e.g., operations 910-930 and the description thereof).

It should be noted that the above description is merely provided for the purposes of illustration, and not intended to limit the scope of the present disclosure. For persons having ordinary skills in the art, multiple variations may be made under the teachings of the present disclosure. However, those variations do not depart from the scope of the present disclosure within the scope of the claims. In some embodiments, the processing device 120 may include one or more additional modules, such as a storage module (not shown) for storing data.

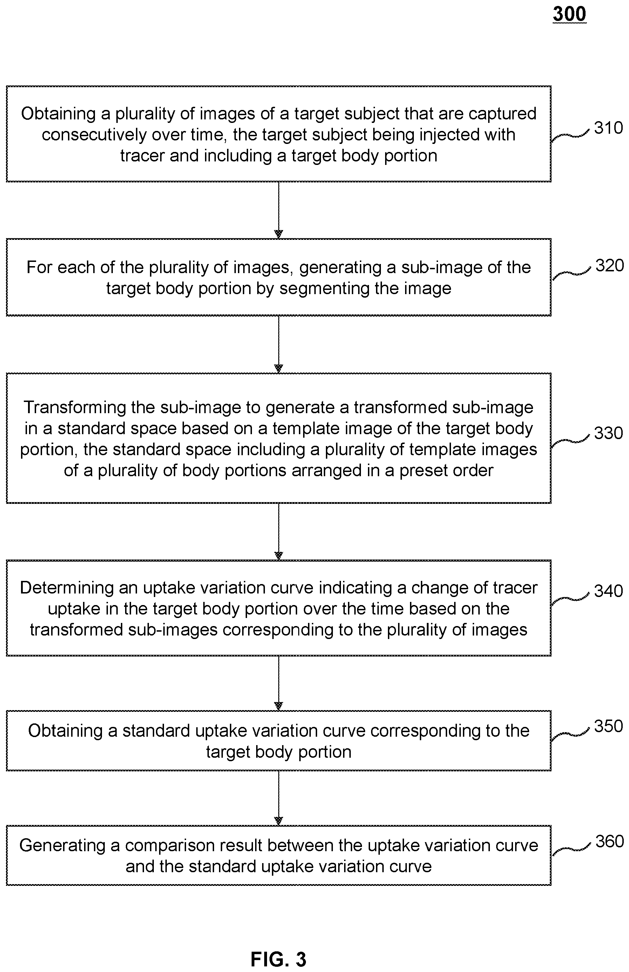

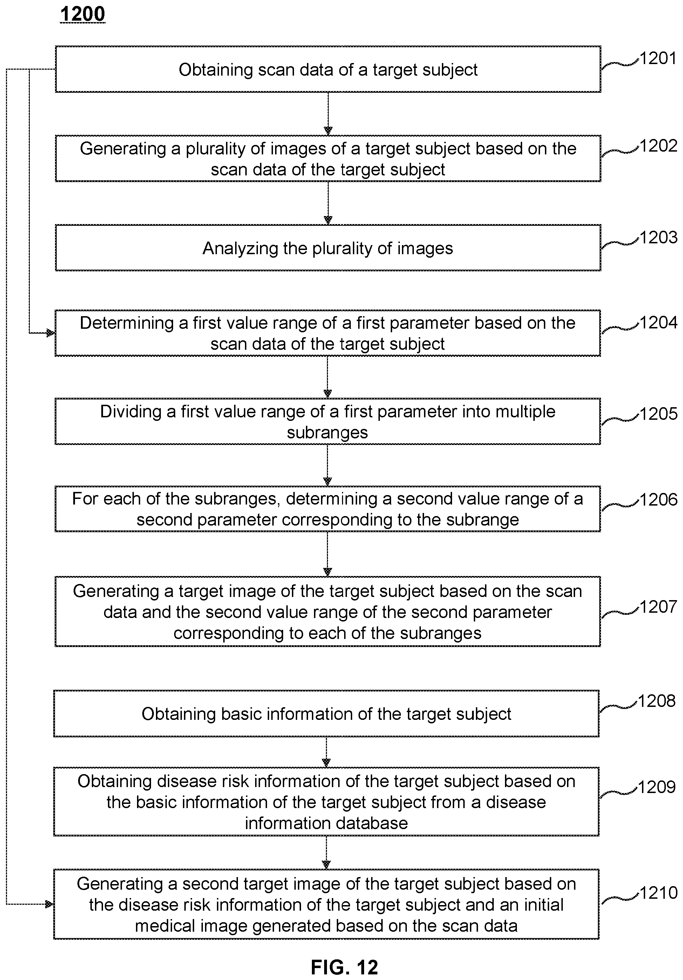

FIG. 3 is a flowchart illustrating an exemplary image analysis process according to some embodiments of the present disclosure. In some embodiments, process 300 may be executed by the medical system 100. For example, the process 300 may be implemented as a set of instructions (e.g., an application) stored in a storage device (e.g., the storage device 130). In some embodiments, the processing device 120 (e.g., one or more modules illustrated in FIG. 2) may execute the set of instructions and may accordingly be directed to perform the process 300. In some embodiments, the process 300 may be performed to achieve at least part of operation 1220 as described in connection with FIG. 12.

In 310, the processing device 120 (e.g., the obtaining module 210) may obtain a plurality of images of a target subject that are captured consecutively over time.

In some embodiments, the target subject may include a human being (e.g., a patient), an animal, or a specific portion, organ, and/or tissue thereof. Merely by way of example, the target subject may include head, chest, abdomen, heart, liver, upper limbs, lower limbs, or the like, or any combination thereof. In the present disclosure, the term "object" or "subject" are used interchangeably in the present disclosure.

In some embodiments, the target subject may include a plurality of body portions. A body portion may be an organ, a tissue, and/or a specific portion of the target subject. Merely by way of example, the target subject may be a human body, and the body portions may include the brain, the liver, the heart, etc. of the human body. In some embodiments, the target subject may include a target body portion. The target body portion may be any one of the plurality of body portions of the target subject that need to be analyzed. In some embodiments, the processing device 120 may designate each of the plurality of body portions of the target subject as the target body portion, and then perform process 300 for each body portion.

In some embodiments, the target subject may be injected with a tracer, and the plurality of images may be multiple frames of PET images of the target subject arranged in time. The tracer may also be referred to as an imaging agent. The tracer may refer to a substance that can be detected by a PET scanner (e.g., the imaging device 110) during a PET scan. In some embodiments, a value of an image element (e.g., a pixel) in each of the plurality of images may indicate tracer uptake of a physical point in the target subject corresponding to the image element. The tracer uptake may relate to at least one of a tracer uptake concentration or a tracer standard uptake value (SUV).

In 320, for each of the plurality of images, the processing device 120 (e.g., the generation module 220) may generate a sub-image of the target body portion by segmenting the image.

A sub-image of the target body portion may also be referred to as a segmentation image of the target body portion. In some embodiments, the processing device 120 may segment the image using an image segmentation algorithm and/or a segmentation network, for example, a convolutional neural network, a recurrent neural network, etc.

In 330, the processing device 120 (e.g., the determination module 230) may transform the sub-image of the target body portion to generate a transformed sub-image of the target body portion in a standard space based on a template image of the target body portion.

The standard space refers to a space that includes standard information, that is, standard sizes and contours of the plurality of body portions of the target subject. Different types of subjects have different standard spaces. The different types of subjects may refer to different species or the same species but have different features (e.g., ages, genders). For example, the different types of subjects may include a human being, a lion, and a tiger. As another example, the different types of subjects may include a minor, an adult, and an old person. In some embodiments, the processing device 120 may obtain a plurality of candidate standard spaces corresponding to the different types of subjects and determine the standard space from the plurality of candidate standard spaces based on a type of the target subject.

The standard space includes a plurality of template images of the plurality of body portions arranged in a preset manner. The preset manner may relate to an arrangement order of the plurality of body portions in subjects. For example, for a standard space corresponding to human beings, the preset manner may relate to an arrangement order of organs, tissues, and specific portions in a human being. Specifically, in the standard space of a human being, the template images of the organs, tissues, and specific portions of the human being may be arranged according to the corresponding positions of the organs, tissues, and specific portions of the human being. Different types of subjects may correspond to different preset manners. The processing device 120 may determine the preset manner based on the type of the target subject. A template image of a body portion may be an image or a model in which the body portion has a standard size and a standard contour.

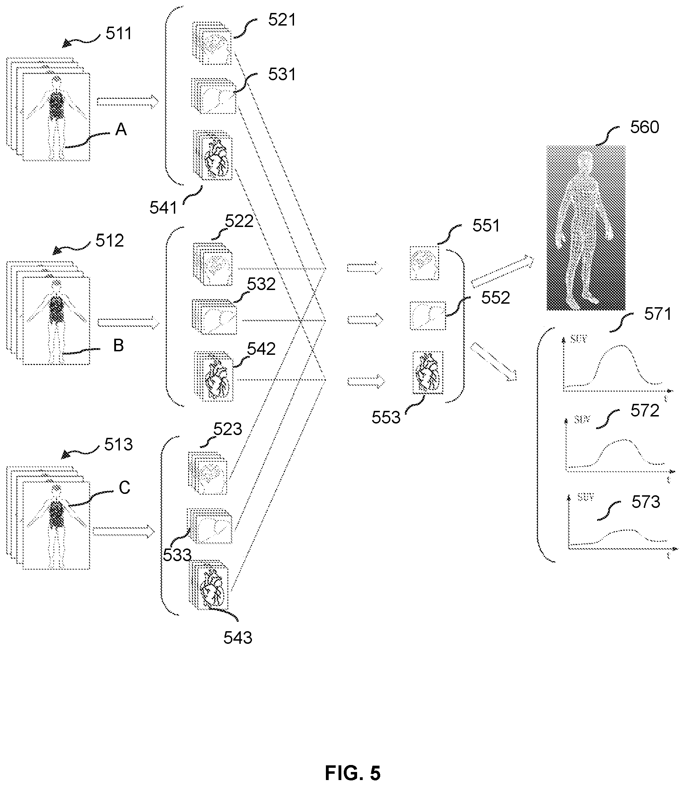

The standard space is generated based on sample images of a plurality of sample subjects. For each of the plurality of sample subjects that are in a preset state and injected with the tracer, the processing device 120 may obtain sample images of the sample subject captured consecutively over time. For each of the plurality of body portions, the processing device 120 may generate segmentation images of the body portion by segmenting the body portion from each of the sample images of each sample subject and generate the template image of the body portion by standardizing the segmentation images of the body portion. Further, the processing device 120 may generate the standard space by arranging the template images of the plurality of body portions. More descriptions regarding the generation of the standard space may be found elsewhere in the present disclosure (e.g., FIG. 5 and the description thereof).

The processing device 120 generates a registered sub-image of the target body portion by registering the sub-image with the template image. In the registration process, the processing device 120 may adjust size information, contour information, and/or position information (e.g., pixel coordinates) of the target body portion in the sub-image based on size information, contour information, and/or position information of the target body portion in the template image, so that the sub-image matches the template image in terms of a size, a contour, and/or a position of the target body portion.

The adjustment during the registration process may lead to the loss of some tracer uptake information. In order to preserve the tracer uptake information, the processing device 120 generates the transformed sub-image by adjusting element values (e.g., pixel values, voxel values) in the registered sub-image based on element values in the sub-image. For example, the processing device 120 may adjust the element values in the registered sub-image and designate the adjusted registered sub-image as the transformed sub-image. In some embodiments, an average, a maximum value, and/or a minimum value of the element values in the transformed sub-image may be consistent with (e.g., the same as) an average, a maximum value, and/or a minimum value of the element values in the sub-image, respectively. Alternatively, the processing device 120 may divide the target body portion into a plurality of regions and designate each of the plurality of regions as a target region. Further, the processing device 120 may make an average, a maximum value, and/or a minimum value of the element values in the target region of the transformed sub-image to be consistent with (e.g., the same as) an average, a maximum value, and/or a minimum value of the element values in a corresponding target region of the sub-image.

In 340, the processing device 120 (e.g., the determination module 230) determines an uptake variation curve indicating a change of tracer uptake in the target body portion over the time based on the transformed sub-images corresponding to the plurality of images.

In some embodiments, an abscissa of the uptake variation curve may represent the time, and an ordinate of the uptake variation curve may represent a parameter for measuring the tracer uptake.

In some embodiments, the processing device 120 may determine a change of a pixel count rate of the target body portion over the time based on the transformed sub-images corresponding to the plurality of images. The pixel count rate of the target body portion may refer to a ratio of a count of target sub-regions of the target body portion that meet a certain condition to a total count of sub-regions of the target body portion. For example, for each transformed sub-image, the processing device 120 may determine the sub-regions of the target body portion by sliding a window with a preset size in the transformed sub-image. When an average pixel value of pixels in the window reaches or exceeds a first preset threshold, the processing device 120 may determine the region in the window as a sub-region of the target body portion. Further, for each sub-region of the target body portion, the processing device 120 may determine whether the average pixel value of pixels in the sub-region reaches or exceeds a second preset threshold. When the average pixel value of pixels in the sub-region reaches or exceeds the second preset threshold, the processing device 120 may determine that the sub-region meets the certain condition and designate the sub-region as a target sub-region of the target body portion.

Different types of body portions may correspond to different preset sizes, different first preset thresholds, and/or different second preset thresholds. The processing device 120 may determine the preset size, the first preset threshold, and/or the second preset threshold based on the type of the target body portion. In some embodiments, the preset size may be the same as or determined based on an empirical size of the target body portion; the first preset threshold may be equal to or slightly less than a minimum empirical pixel value of the target body portion; and the second preset threshold may be equal to or slightly larger than a maximum empirical pixel value of the target body portion.

The processing device 120 may determine a change of the tracer uptake of the target body portion over the time based on the change of the pixel count rate and a preset calibration coefficient. The preset calibration coefficient may be determined based on a device (e.g., the imaging device 110) that captures the plurality of images. Different devices may correspond to different preset calibration coefficients. Merely by way of example, the tracer uptake concentration may be equal to a product of the change of the pixel count rate and preset calibration coefficient. As another example, the SUV may be equal to the tracer uptake concentration divided by a ratio of a concentration of the tracer to a weight of the target subject. Further, the processing device 120 may generate the uptake variation curve corresponding to the target body portion based on the change of the tracer update of the target body portion over time.

In 350, the processing device 120 (e.g., the obtaining module 210) may obtain a standard uptake variation curve corresponding to the target body portion. The standard uptake variation curve may indicate a change of tracer uptake in the target body portion of a subject when vital signs of the subject are maintained at a normal level.

In some embodiments, different tracers or different body portions may correspond to different standard uptake variation curves. The processing device 120 may obtain the standard uptake variation curve corresponding to the target body portion based on the type of the target body portion and the type of the tracer injected into the target subject. For example, if the target body portion is the liver and the injected tracer is K, the processing device 120 may obtain a standard uptake variation curve corresponding to the liver and the tracer K.

In some embodiments, various standard uptake variation curves corresponding to various body portions and/or various tracers may be previously determined and stored in a storage device (e.g., the storage device 130) disclosed elsewhere in the present disclosure and/or an external storage device. The processing device 120 may obtain the standard uptake variation curve corresponding to the target body portion from the storage device and/or the external storage device via a network (e.g., the network 150). More descriptions regarding the standard uptake variation curve may be found elsewhere in the present disclosure (e.g., FIG. 5 and the description thereof).

In 360, the processing device 120 (e.g., the generation module 220) may generate a comparison result between the uptake variation curve and the standard uptake variation curve.

In some embodiments, the comparison result may include at least one of a comparison graph indicating the uptake variation curve and the standard uptake variation curve or difference information relating one or more parameters of the uptake variation curve and the standard uptake variation curve. The one or more parameters may include a time point when a curve (e.g., the uptake variation curve, the standard uptake variation curve) begins to rise or fall, a ring speed of the curve, a falling speed of the curve, a peak value of the curve; a magnitude of a change of the curve in a preset period, or the like, or any combination thereof. According to or by analyzing the comparison result, the blood flow and metabolism of the current state of the target subject relative to the preset state for generating the standard uptake variation curve may be obtained. For example, if the uptake variation curve and the standard uptake variation curve are very different (e.g., having a difference exceeding a threshold) in shape or parameters, the blood flow and metabolism of the current state of the target subject are abnormal.

According to the embodiments of the present disclosure, the sub-images of the target body portion may be transformed into the standard space, which may reduce the impact of differences in the morphology of the target body portions of different target subjects on the analysis of blood flow and metabolism, thereby improving the evaluation accurate of the blood flow and metabolism. In addition, by the registration process, morphological differences of the target body portion in the sub-image and the corresponding template image are reduced, and further, by adjusting the element values in the registered sub-image, the tracer uptake information of the target body portion is preserved, which may further improve the evaluation accurate of the blood flow and metabolism.

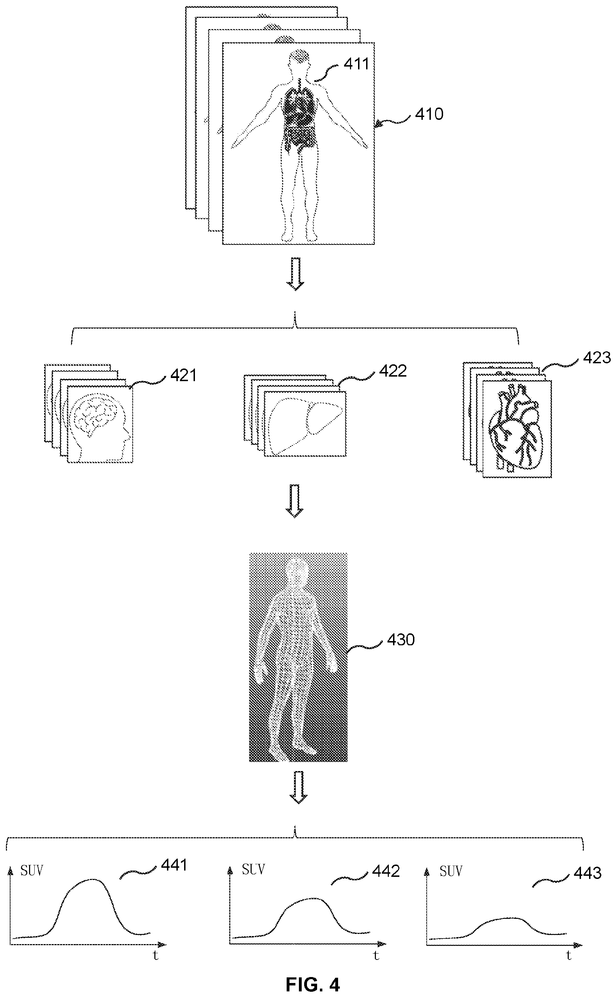

FIG. 4 is a schematic diagram illustrating an exemplary image analysis process according to some embodiments of the present disclosure.

In some embodiments, for each of a plurality of images of a target subject that are captured consecutively over time, the processing device 120 may segment the image to generate a plurality of sub-images of multiple body portions of the target subject. Each of the plurality of sub-images may correspond to one of the body portions of the target subject. For example, as shown in FIG. 4, the processing device 120 segments each of a plurality of images 410 of a patient 411 to generate a first sub-image 421 corresponding to the brain, a second sub-image 422 corresponding to the liver, and a third sub-image 423 corresponding to the heart. After the plurality of images 410 are segmented, a plurality of first sub-images 421 of the brain, a plurality of second sub-images 422 of the liver, and a plurality of third sub-images 423 of the heart are generated.

The processing device 120 may transform each first sub-image 421 to generate a first transformed sub-image of the brain in a standard space 430 based on a template image of the brain, transform each second sub-image 422 to generate a second transformed sub-image of the liver in the standard space 430 based on a template image of the liver, and transform each third sub-image 423 to generate a third transformed sub-image of the heart in the standard space 430 based on a template image of the heart. After the plurality of first sub-images 421, the plurality of second sub-images 422, and the plurality of third sub-images 423 are transformed, a plurality of first transformed sub-images of the brain, a plurality of second transformed sub-images of the liver, and a plurality of third transformed sub-images of the heart are generated. Further, the processing device 120 may determine an uptake variation curve 441 indicating a change of tracer uptake (e.g., SUV) in the brain over the time based on the plurality of first transformed sub-images, an uptake variation curve 442 indicating a change of the tracer uptake in the liver over the time based on the plurality of second transformed sub-images, and an uptake variation curve 443 indicating a change of the tracer uptake in the heart over the time based on the plurality of third transformed sub-images.

FIG. 5 is a schematic diagram illustrating an exemplary process for determining a standard space and a standard uptake variation curve according to some embodiments of the present disclosure.

The standard space is generated based on sample images of a plurality of sample subjects that are in a preset state and injected with a tracer. Specifically, for each sample subject, the processing device 120 obtains sample images of the sample subject captured consecutively over time (e.g., multiple sample PET images of the sample subject obtained in a PET scan). In some embodiments, the sample subjects may be of a same type as the target subject. For instance, the sample subjects and the target subject may both be human beings. Optionally, the sample subjects and the target subject may be different human beings in a same age range (e.g., younger than 10 years old, 11-18 years old, 19-60 years old, older than 60 years old).

The preset state may refer to a state in which vital signs of the sample subjects are maintained at a preset level. For example, the sample subjects have normal metabolic status, no disability, and no diseased organs or tissues. As shown in FIG. 5, the processing device 120 obtain sample images 511 of a sample subject A, sample images 512 of a sample subject B, and sample images 513 of a sample subject C.

For each of a plurality of body portions, the processing device 120 generates segmentation images of the body portion by segmenting the body portion from each of the sample images of each sample subject. For example, as shown in FIG. 5, the processing device 120 may segment the brain, the liver, and the heart from the sample images 511 to generate segmentation images 521 of the brain, segmentation images 531 of the liver, and segmentation images 541 of the heart, respectively. The processing device 120 may segment the brain, the liver, and the heart from the sample images 512 to generate segmentation images 522 of the brain, segmentation images 532 of the liver, and segmentation images 542 of the heart, respectively. The processing device 120 may segment the brain, the liver, and the heart from the sample images 513 to generate segmentation images 523 of the brain, segmentation images 533 of the liver, and segmentation images 543 of the heart, respectively.

For each of the plurality of body portions, the processing device 120 generates a template image of the body portion by standardizing the segmentation images of the body portion. Specifically, the processing device 120 may extract size information and contour information of the body portion from each of the segmentation images of the body portion. The size information may include area and/or volume of the body portion. The contour information may be related to a type of the body portion. For example, the contour information of the brain may include a length-width ratio of the brain; the contour information of the liver may include a left-right diameter, an upper-lower diameter, and a front-to-back diameter of the liver; the contour information of the heart may include a long diameter, a wide diameter, and a front-to-back diameter of the heart; the contour information of the eyeball may include a long axis and a short axis of the eyeball.

The processing device 120 determines a standard size and a standard contour of the body portion based on the size information and contour information of the body portion extracted from each segmentation image of the body portion. In some embodiments, the processing device 120 may determine an average or a weighted average of the size information of the body portion as the standard size of the body portion, and determine an average or a weighted average of the contour information of the body portion as the standard contour of the body portion. The processing device 120 generates the template image of the body portion based on the standard size and the standard contour of the body portion. As shown in FIG. 5, the processing device 120 may generate a template image 551 of the brain by standardizing the segmentation images 521, 522, and 523 of the brain; a template image 552 of the liver by standardizing the segmentation images 531, 532, and 533 of the liver; and a template image 553 of the heart by standardizing the segmentation images 541, 542, and 543 of the heart.

Further, the processing device 120 generates the standard space by arranging the template images of the plurality of body portions in a preset manner as described in connection with FIG. 3. As shown in FIG. 5, the processing device 120 may generate a standard space 560 by arranging the template image 551 of the brain, the template image 552 of the liver, and of the template image 553 of the heart according to the corresponding positions of the brain, the liver, and the heart in a human being.

In some embodiments, the processing device 120 may generate a standard uptake variation curve of each of the plurality of body portions. An abscissa of the standard uptake variation curve may represent the time, and an ordinate of the standard uptake variation curve may represent a parameter for measuring the tracer uptake (e.g., the tracer uptake concentration, the SUV).

In some embodiments, the determination of the standard uptake variation curve may be similar to the determination of the uptake variation curve as described in connection with FIG. 3. Specifically, for each of the plurality of sample subjects, the processing device 120 may determine a change of a pixel count rate of the body portion of the sample subject over time based on the segmentation images of the body portion of the sample subject. The processing device 120 may determine a change of a tracer uptake of the body portion of the sample subject over time based on a change of the pixel count rate and a preset calibration coefficient. Further, the processing device 120 may generate a standard uptake variation curve corresponding to the body portion based on the change of the tracer update of the body portion of each sample subject. As shown in FIG. 5, the processing device 120 may generate a standard uptake variation curve 571 corresponding to the brain, a standard uptake variation curve 572 corresponding to the liver, and a standard uptake variation curve 573 corresponding to the heart.

In some embodiments, data related to a standard space and/or standard uptake variation curves of body portions in the standard space may be stored in a hash table in a storage device (e.g., the storage device 130 or an external storage device). The hash table may include multiple key-value pairs. The key in a key-value pair may be used to store an index (e.g., an identifier) of a template image of a body portion, and the value in the key-value pair may be used to store information (e.g., size information, contour information, position information) of the template image and the information (e.g., abscissa values, ordinate values) of the standard uptake variation curve of the body portion.

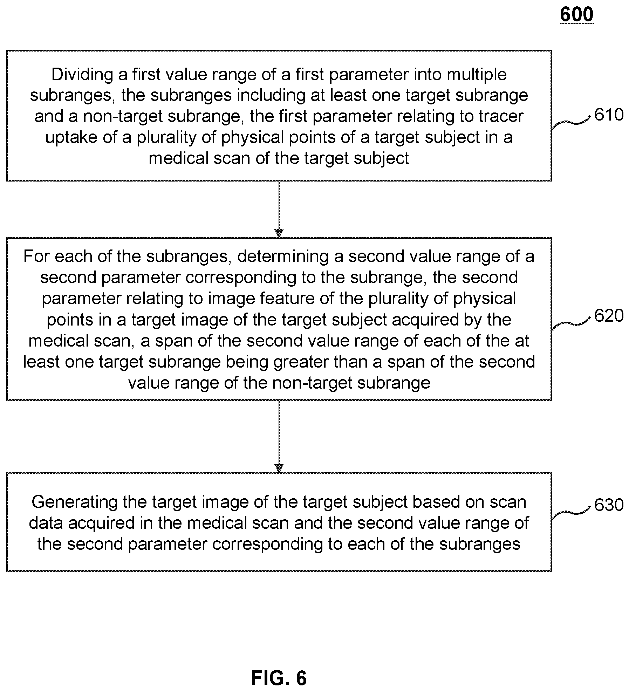

FIG. 6 is a flowchart illustrating an exemplary image display process according to some embodiments of the present disclosure. In some embodiments, process 600 may be executed by the medical system 100. For example, the process 600 may be implemented as a set of instructions (e.g., an application) stored in a storage device (e.g., the storage device 130). In some embodiments, the processing device 120 (e.g., one or more modules illustrated in FIG. 2) may execute the set of instructions and may accordingly be directed to perform the process 600. In some embodiments, the process 600 may be performed to achieve at least part of operation 1230 as described in connection with FIG. 12.

In 610, the processing device 120 (e.g., the determination module 230) may divide a first value range of a first parameter into multiple subranges.

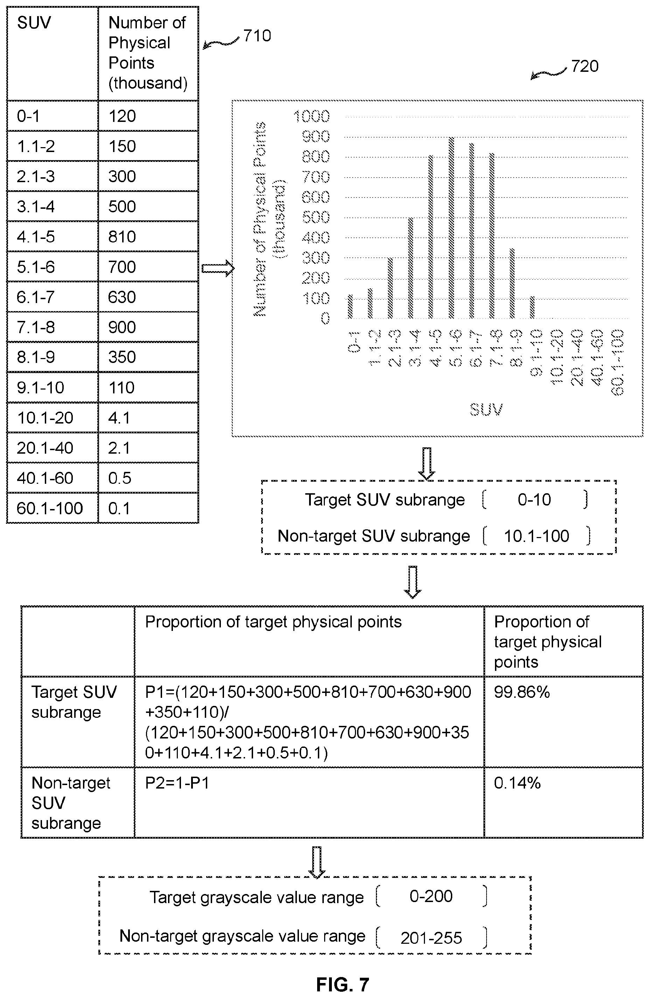

The first parameter may relate to tracer uptake of a plurality of physical points of a target subject in a medical scan (e.g., a PET scan) of the target subject. In some embodiments, the first parameter may include at least one of a tracer uptake concentration or an SUV. The processing device 120 may determine the first value range of the first parameter based on values of the first parameter of the plurality of physical points, wherein the values of the first parameter (e.g., SUV values) may be determined based on PET data collected in the PET scan. In some embodiments, the processing device 120 may determine a maximum value and a minimum value among the values of the first parameter of the plurality of physical points, and then designate a range from the minimum value to the maximum value as the first value range. For example, as shown in FIG. 7, a maximum SUV and a minimum SUV of physical points in Table 710 may be 100 and 0, respectively, and accordingly, a first value range of the SUV may be 0-100.

The subranges may include at least one target subrange and a non-target subrange. In some embodiments, physical points corresponding to values in the non-target subrange usually have well-defined properties that can be evaluated without analysis, and physical points corresponding to values in the at least one target subrange usually have ambiguous properties that need to be evaluated by further analysis.

Normally, high values (e.g., SUV 10-100) of the first parameter correspond to some organs with higher tracer uptake (e.g., regions of high normal metabolism) or larger lesions that are easy to be identified, and while small values (e.g., SUV 2.5-10) of the first parameter correspond to suspicious lesions that are not much different in the first parameter from normal organs or tissues (usually with SUV 0-10) and difficult to be identified. Therefore, physical points with high values of the first parameter may not need to be analyzed in detail, while physical points with small values of the first parameter may need to be further evaluated and analyzed. The at least one target subrange may include the small values of the first parameter, and the non-target subrange may include the high values of the first parameter. As used herein, the small values of the first parameter may refer to values of the first parameter less than a preset threshold (e.g., SUV 10), and the high values of the first parameter may refer to values of the first parameter larger than the preset threshold.

Traditionally, the first value range (e.g., 0-100) of the first parameter is not divided, and values of the first parameter in the first value range are transformed to values in a preset grayscale value range (e.g., 0-255) or a color scale range by performing, for example, a linear transformation. For example, both small values of the first parameter (e.g., SUV 0-10) and the high values of the first parameter (e.g., SUV 10.1-100) are transformed to values in the grayscale value range 0-255. However, a count of physical points with high values of the first parameter is usually much less than a count of physical points with small values of the first parameter. For example, in the target subject, SUV values of 99% of physical points are in 0-10, and SUV values of 1% of physical points are in 10.1-100. Therefore, the above traditional method may cause that grayscale values of the physical points with high values of the first parameter are larger than grayscale values in other regions in the generated target image, and grayscale values of the physical points with small values of the first parameter are very close to each other in the generated target image.

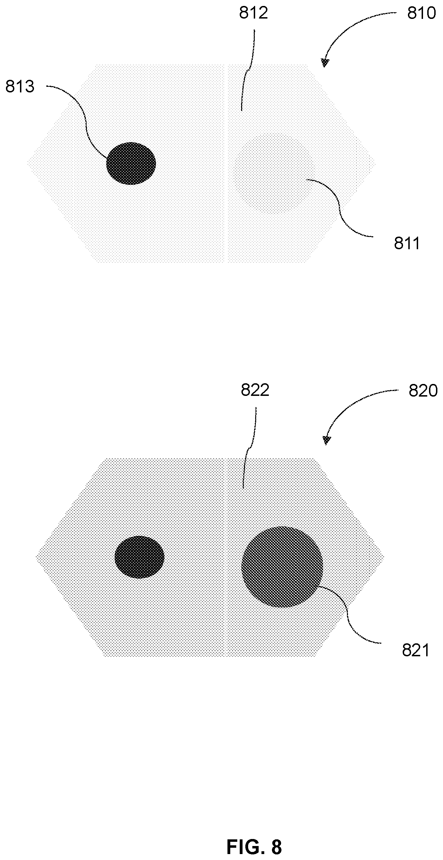

For example, as shown in FIG. 8, in an image 810 generated by the above traditional method, a region 811 corresponds to physical points with small values of the first parameter, a region 813 corresponds to physical points with high values of the first parameter. Grayscale values of the region 811 are very close to a normal region 812 of the image 810, and grayscale values of the region 813 are larger than grayscale values of the normal region 812. In such cases, it is difficult to identify the suspicious lesions corresponding to the small values of the first parameter from the image 810, thereby resulting in the poor readability of the image 810.

In the present disclosure, the processing device 120 may divide the first value range of the first parameter into the multiple subranges and further determine a value range of an image feature (e.g., a grayscale value or a color scale value) corresponding to each of the subranges. If the value of the first parameter of a physical point is in a specific subrange of the first parameter, the value of the image feature (e.g., the grayscale value) of this physical point may be determined by transforming the value of the first parameter into a corresponding value range of the image feature.

In some embodiments, the processing device 120 may determine a value distribution of the first parameter in the first value range based on values of the first parameter of the plurality of physical points, and divide the first value range of the first parameter into the multiple subranges based on the value distribution. For example, the value distribution of the first parameter in the first value range may indicate the number of physical points corresponding to each of multiple sub-ranges of the first parameter. Then, the processing device 120 may determine one or more division values based on the value distribution. For example, if the number of physical points corresponding to sub-ranges larger than a specific value has a large difference from the number of physical points corresponding to sub-ranges smaller than the specific value, the value may be designated as a division point. As another example, if the number of physical points corresponding to sub-ranges larger than a specific value only accounts for a small proportion among all the physical points, the specific value may be designated as a division point. As yet another example, if the number of physical points corresponding to sub-ranges larger than a specific value is higher than a preset threshold, while the number of physical points corresponding to sub-ranges smaller than the specific value is smaller than a preset threshold, the specific value may be designated as a division value.

For example, as shown in FIG. 7, by counting SUVs of the physical points in the Table 710, the processing device 120 may determine an SUV distribution 720 in the first value range of 0-100. According to the SUV distribution 720, the processing device 120 may determine that 10 is a division value because only a small number of physical points have SUVs greater than 10 (i.e., the number of physical points corresponding to subranges lager than 10 has a large difference from the number of physical points corresponding to subranges smaller than 10). After the division value is determined, a value range (e.g., 0-10) from the minimum value of the first parameter to the division value may be designated as a target SUV subrange, and a value range (e.g., 10.1-100) from the division value to the maximum value of the first parameter may be designated as a non-target SUV subrange 10.1-100. In such cases, the number of physical points corresponding to the non-target SUV subrange may only account for a small proportion among all the physical points. In some embodiments, multiple division values may be determined, and the processing device 120 may determine a plurality of target SUV subranges. For example, the processing device 120 may further determine that 2 is another SUV division value, and the value range of the SUV may be divided into a target SUV subrange 0-2, a target SUV subrange 2.1-10, and a non-target SUV subrange 10.1-100.

In some embodiments, the processing device 120 may divide the first value range of the first parameter into the multiple subranges based on preset ranges or at least one preset division value. For example, the preset ranges or the at least one preset division value may be specified by a user (e.g., a medical staff). For example, preset ranges may include an SUV range 0-10 and an SUV range 10-100, or the preset division value may be 10. In such cases, an SUV range 0-100 (i.e., the first value range) may be divided into a target SUV subrange 0-10 and a non-target SUV subrange 10.1-100.

In 620, for each of the subranges, the processing device 120 (e.g., the determination module 230) may determine a second value range of a second parameter corresponding to the subrange.

The second parameter may relate to image feature of the plurality of physical points in a target image of the target subject acquired by the medical scan. In some embodiments, the image feature may include at least one of a grayscale value or a color scale value.

In some embodiments, for a physical point of the target subject, the value of the first parameter is determined based on scan data acquired in the medical scan of the target subject, and the value of the first parameter may reflect the metabolism of the physical point of the target subject. In order to display the physical point in an image, the value of the first parameter of the physical point may need to be transformed into the value of the second parameter of the physical point (which relates to the display form of the physical point). Specifically, in the present disclosure, second value ranges corresponding to different subranges of the first parameter are determined, and if the value of the first parameter of the physical point is in a specific subrange, the value of the first parameter is transformed into the second value range corresponding to the specific subrange to determine the value of the second parameter of the physical point.

In some embodiments, for each of the subranges, the processing device 120 may determine target physical points among the plurality of physical points. A value of the first parameter of each target physical point is in the subrange. Further, the processing device 120 may determine a proportion of the target physical points among the plurality of physical points and determine the second value range of the second parameter corresponding to the subrange based on the proportion. Specifically, the larger the proportion, the larger a span of the second value range of the second parameter corresponding to the subrange. In some embodiments, as described in connection with operation 610, a count of physical points (i.e., the physical points corresponding to the at least one target subrange) with high values of the first parameter is usually much less a count of physical points (i.e., the physical points corresponding to the non-target subrange) with small values of the first parameter. Accordingly, the span of the second value range of each of the at least one target subrange is greater than the span of the second value range of the non-target subrange. In this way, physical points with values of the first parameter in the non-target subrange may not affect the display of other physical points with values of the first parameter in the at least one target sub-range, and physical points with values of the first parameter in the at least one target sub-range differentially.

As shown in FIG.7, a count of target physical points corresponding to the target SUV subrange 0-10 is 4930 thousand, and a count of all physical points is 4936.8 thousand. Therefore, a proportion P1 of the target physical points corresponding to the target SUV subrange 0-10 is about 99.86%, and a proportion P2 of target physical points corresponding to the non-target SUV subrange 101-100 is (1-P1)=0.14%. Accordingly, a span of the second value range corresponding to the target SUV subrange 0-10 should be much larger than a span of the second value range corresponding to the non-target SUV subrange 10.1-100. For example, as shown in FIG.7, a target grayscale value range (i.e., the second value range) corresponding to the target SUV subrange 0-10 is 0-200, and a non-target grayscale value range corresponding to the non-target SUV subrange 10.1-100 is 201-255. In some embodiments, the second value range corresponding to the target SUV subrange 0-10 may be a first color scale range, and the second value range corresponding to the non-target SUV subrange 10.1-100 may be a second color scale range. For example, the first color scale range may be a red color scale range, and the red color scales in the red color scale range become darker as the SUVs increase in the target SUV subrange 0-10. The second color scale range may be blue color scale range, and the blue color scales in the blue color scale range become darker as the SUV increases in the non-target SUV subrange 10.1-100.

In some embodiments, values of the second parameter in the second value range may be determined by performing a linear transformation or a non-linear transformation on values in the corresponding subrange. The linear transformation may be performed based on a linear function, and the non-linear transformation may be performed based on a non-linear function (e.g., an exponential function, a logarithmic function, etc.). For example, for each value in each subrange, the processing device 120 may determine a corresponding value of the second parameter in the second value range by performing a linear transformation or a non-linear transformation on the value in the subrange. Merely by way of example, for an SUV 2 in the target SUV subrange 0-10, the processing device 120 may determine a grayscale value 40 in the second value range 0-200 by performing the linear transformation on the SUV 2, and for an SUV 2.5 in the target SUV subrange 0-10, the processing device 120 may determine a grayscale value 50 in the second value range 0-200 by performing the linear transformation on the SUV 2.5.

In 630, the processing device 120 (e.g., the generation module 220) may generate the target image of the target subject based on scan data acquired in the medical scan and the second value range of the second parameter corresponding to each of the subranges.

For example, for each physical point of the target subject, the processing device 120 may determine an SUV of the physical point based on the scan data, determine a subrange corresponding to the SUV, and determine a grayscale value based on the SUV and the second value range corresponding to the determined subrange. The target image may include a pixel or voxel with the determined grayscale value corresponding to the physical point.

In the present disclosure, the first value range (e.g., 0-100) of the first parameter is divided into the at least one target subrange and the non-target subrange, and the span of the second value range of each of the at least one target subrange is greater than the span of the second value range of the non-target subrange. Therefore, in the generated target image, grayscale value range (or color scale value range) corresponding to physical points with values in the at least one target subrange is greater than grayscale value range (or color scale value range) corresponding to physical points with values in the non-target subrange, accordingly the contrast of a region of the generated target image corresponding to the at least one target subrange is improved. For example, according to the above traditional method, a grayscale value determined by performing the linear transformation on the SUV 2 is 5, and a grayscale value determined by performing the linear transformation on the SUV 2.5 is 6. According to the image display method (e.g., operations 610-630) in the present disclosure, a grayscale value determined by performing the linear transformation on the SUV 2 is 40, and a grayscale value determined by performing the linear transformation on the SUV 2.5 is 50. In image display, the visual difference between a pixel with a grayscale value of 50 and a pixel with a grayscale value of 40 is greater than the visual difference between a pixel with a grayscale value of 5 and a pixel with a grayscale value of 6. Therefore, by the image display method in the present disclosure, the details in the region of the target image corresponding to the at least one target subrange are clear, which facilitates the identification of the suspicious lesions. For example, as shown in FIG. 8, in an image 820 generated by the image display method in the present disclosure, grayscale values of a region 821 corresponding to physical points with small values of the first parameter (i.e., the target subrange) are larger than grayscale values of a normal region 822.

In some embodiments, in response to an instruction to zoom-in a target region of the target image, the processing device 120 may perform image interpolation (e.g., a linear interpolation) on target region of the target image, thereby improving the image clarity. In some embodiments, if an SUV range corresponding to the target region is 5-100, a grayscale value range corresponding to the SUV range 5-100 determined by the traditional method is 12-255. In the grayscale value range 12-255, the maximum grayscale value is nearly 20 times the minimum grayscale value, so the error of the image interpolation is relatively high. A grayscale value range corresponding to the SUV range 5-100 determined by the image display method in the present disclosure is 100-255. In the grayscale value range 100-255, the maximum grayscale value is only 2.55 times the minimum grayscale value, so the error of the image interpolation is relatively small, thereby improving the clarity of the zoomed-in target image.

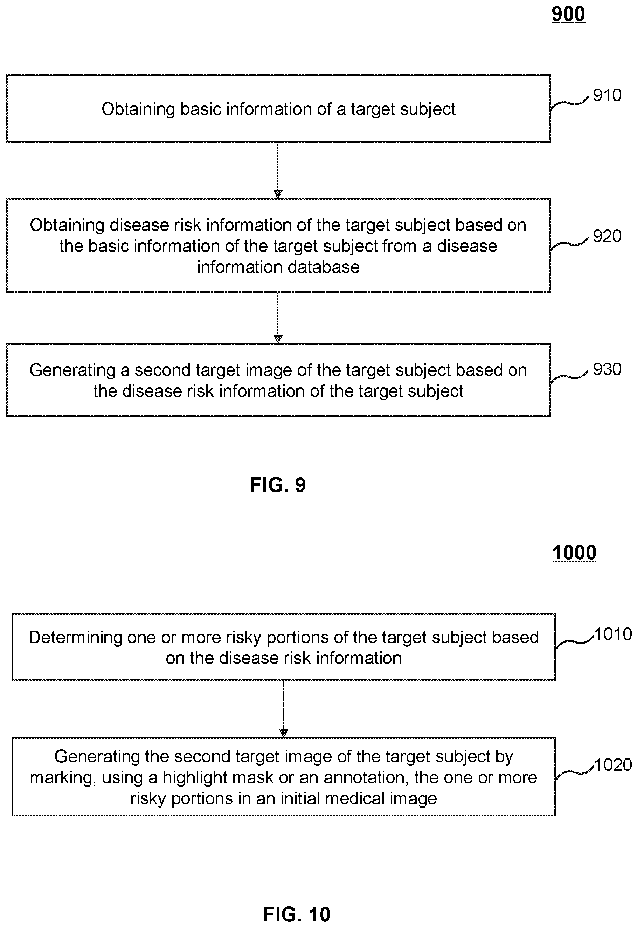

FIG. 9 is a flowchart illustrating an exemplary image display process according to some embodiments of the present disclosure. In some embodiments, process 900 may be executed by the medical system 100. For example, the process 900 may be implemented as a set of instructions (e.g., an application) stored in a storage device (e.g., the storage device 130). In some embodiments, the processing device 120 (e.g., one or more modules illustrated in FIG. 2) may execute the set of instructions and may accordingly be directed to perform the process 900. In some embodiments, the process 900 may be performed to achieve at least part of operation 1230 as described in connection with FIG. 12.

In 910, the processing device 120 (e.g., the obtaining module 210) may obtain basic information of a target subject.

The basic information may refer to information related to the target subject. In some embodiments, the basic information of the target subject may include at least one of a name, ID card information, medical insurance information, medical card information, or medical record information. Exemplary ID card information may include ID number, gender, date of birth, native place, etc. Exemplary medical insurance information may include medical insurance number, medical insurance usage records, medical insurance coverage, etc. Exemplary medical card information may include the number of the medical card, the name and ID number of the cardholder, etc. Exemplary medical record information may include historical medical records, historical surgical plans, historical prescriptions, historical medical images, etc. In some embodiments, the processing device 120 may uniquely identify the target subject based on the basic information of the target subject. That is, the processing device 120 can identify the target subject from multiple subjects based on the basic information of the target subject.

In some embodiments, the processing device 120 may obtain the basic information of the target subject by using a scanner, a sensor, etc. to scan the target subject's ID card, medical insurance card, etc. Alternatively, the processing device 120 may obtain the basic information of the target subject based on an input of the target subject. In some embodiments, the basic information of the target subject may be previously obtained and stored in a storage device (e.g., the storage device 130) disclosed elsewhere in the present disclosure and/or an external storage device. The processing device 120 may obtain the basic information from the storage device and/or the external storage device via a network (e.g., the network 150).

In 920, the processing device 120 (e.g., the obtaining module 210) may obtain disease risk information of the target subject based on the basic information of the target subject from a disease information database.

The disease information database may refer to a database used to store basic information and disease risk information of a plurality of subjects. In some embodiments, the basic information and the disease risk information of the plurality of subjects may be previously obtained and stored in the disease information database.

The disease risk information of a subject may indicate whether the subject has a high risk of disease. In some embodiments, the disease risk information may include risk gene information, disease history information, family genetic disease information, and bad habit information. The risk gene information may refer to information related to disease-causing genes that the target subject may carry. For example, the risk gene information may include an onset site of a disease caused by the gene, a type of the disease caused by the gene, an onset probability of the disease caused by the gene, etc. The disease history information may refer to information related to the target subject's disease history. For example, the disease history information may include an onset site, an onset time, a treatment plan, etc. of the target subject's historical disease. The family genetic disease information may refer to information related to genetic diseases suffered by relatives of the target subject. For example, the family genetic disease information may include a relationship between the diseased relative and the target subject, and an onset site, an onset time, a treatment plan of the genetic disease, etc. The bad habit information may refer to information related to the target subject's potentially disease-causing living habits. For example, the bad habit information may include bad habit, such as smoking, chewing betel nuts, staying up late, drinking alcohol, etc., and an onset site of disease that these bad habits may cause.

In the disease information database, the basic information and the disease risk information of each of the plurality of subjects may be stored correspondingly. The processing device 120 may retrieve the disease risk information of the target subject based on the basic information of the target subject.

In 930, the processing device 120 (e.g., the generation module 220) may generate a second target image of the target subject based on the disease risk information of the target subject.

The second target image of the target subject may refer to a medical image obtained after processing an initial medical image of the target subject. The initial medical image of the target subject may refer to a medical image generated based on scan data acquired in a medical scan of the target subject. Merely by way of example, the processing may include marking the initial medical image of the target subject using, for example, a highlight mask or an annotation.

In some embodiments, the processing device 120 may determine one or more risky portions of the target subject based on the disease risk information and generate the second target image of the target subject by marking, using the highlight mask or the annotation, the one or more risky portions in the initial medical image. More descriptions regarding the generation of the second target image may be found elsewhere in the present disclosure (e.g., FIG. 10 and the description thereof).

In some embodiments, the processing device 120 may update the disease information database based on the second target image of the target subject and a corresponding diagnostic result of the target subject. The diagnostic result may refer to a result given by a doctor according to the second target image and/or face-to-face consultation of the target subject. Merely by way of example, the diagnostic result may include the name, age, ID number, medical insurance card number, diagnosis process, disease type, treatment plan, etc. of the target subject. For example, the processing device 120 may update disease risk information of the target subject based on the diagnosis process, disease type, treatment plan, of the target subject. As another example, the processing device 120 may determine whether the name, age, ID number, and medical insurance card number in the diagnostic result have been included in the basic information of the target subject. If any of the name, age, ID number, or medical insurance card number in the diagnostic result is not included in the basic information of the target subject, the processing device 120 may update the basic information of the target subject.

In the present disclosure, the second target image of the target subject may be automatically generated based on the disease risk information of the target subject, which does not require human intervention and improves the efficiency, rationality, and quality of the display of the second target image.

FIG. 10 is a flowchart illustrating an exemplary process for generating a second target image according to some embodiments of the present disclosure. In some embodiments, the process 1000 may be performed to achieve at least part of operation 930 as described in connection with FIG. 9.

In 1010, the processing device 120 (e.g., the determination module 230) may determine one or more risky portions of the target subject based on the disease risk information.

The one or more risky portions may refer to organs and/or tissues (e.g., lungs, heart, liver, muscles, nerves, lymph) in the target subject that are at risk of disease. In some embodiments, the processing device 120 may obtain one or more onset sites in the disease risk information and designate the one or more onset sites as the one or more risky portions of the target subject.

In 1020, the processing device 120 (e.g., the generation module 220) may generate the second target image of the target subject by marking, using a highlight mask or an annotation, the one or more risky portions in an initial medical image.

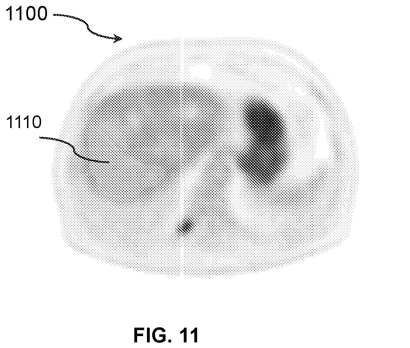

The highlight mask may be an image layer in which a partial region (e.g., regions corresponding to the one or more risky portions) has specific image parameters while the other portions are transparent or have a background value. The processing device 120 may generate the second target image by overlapping the highlight mask on the initial medical image. Merely by way of example, the image parameters may include contrast, brightness, grayscale, saturation, etc. FIG. 11 is a schematic diagram illustrating an exemplary second target image according to some embodiments of the present disclosure. As shown in FIG. 11, a risky portion 1110 in a second target image 1100 may be marked using a highlight mask.

The annotation may be used to make the partial region of the initial medical image by adding, for example, text, lines, borders, etc. The processing device 120 may annotate information (e.g., name of each risky portion, a type of risky disease, and an onset probability of the risky disease, etc.) of the one or more risky portions in the regions of the initial medical image corresponding to the one or more risky portions.

In some embodiments, the processing device 120 may mark different risky portions in different ways. For example, when there are multiple risk portions, such as ovaries and lungs, the processing device 120 may use the highlight mask to mark a region where the ovaries are located and use the annotation to mark a region where the lungs are located.

In some embodiments, the processing device 120 may mark different risky portions using different contrasts. For example, the processing device 120 may use different contrasts to mark the region where the ovaries are located and the region where the lungs are located.

Through the above-mentioned embodiments, the risky portions may be marked in the second target image of the target subject, which may improve the display effect of the second target image and remind the doctor to check the risky portions, thereby improving the diagnostic accuracy of the target subject. In addition, multiple risk portions of the target subject may be marked differently, which may avoid confusion between different risk portions, thereby improving the display effect of the second target image.

In some embodiments, the processing device 120 may generate the second target image of the target subject by marking regions of the initial medical image other than the one or more risky portions.

In some embodiments, the processing device 120 may display the second target image of the target subject via a display interface connected to and/or communicate with the processing device 120.

In some embodiments, the processing device 120 may generate a reminder for the one or more risky portions to remind a user (e.g., the doctor). Merely by way of example, the reminder may be a voice reminder.

In some embodiments, the processing device 120 may construct the disease information database to store the basic information and the disease risk information of each of the plurality of subjects in an associated manner, which may facilitate subsequent queries.

In some embodiments, the user may query information related to the one or more risky portions in the disease information database. For example, after receiving a query request related to a risky portion, the processing device 120 may obtain and display the basic information and the disease risk information of the target subject and/or basic information and the disease risk information of other subjects that have the same risky portion.

FIG. 12 is a flowchart illustrating an exemplary image processing process according to some embodiments of the present disclosure. In some embodiments, process 1200 may be executed by the medical system 100. For example, the process 1200 may be implemented as a set of instructions (e.g., an application) stored in a storage device (e.g., the storage device 130). In some embodiments, the processing device 120 (e.g., one or more modules illustrated in FIG. 2) may execute the set of instructions and may accordingly be directed to perform the process 1200.

In 1201, the processing device 120 (e.g., the obtaining module 210) may obtain scan data of a target subject.

In some embodiments, the processing device 120 may direct or cause the imaging device 110 to scan the target subject to obtain the scan data of the target subject.

In 1202, the processing device 120 (e.g., the obtaining module 210) may generate a plurality of images of a target subject based on the scan data of the target subject. For example, the processing device 120 may generate the plurality of images of the target subject by reconstructing the scan data of the target subject.

In 1203, the processing device 120 (e.g., the obtaining module 210, the generation module 220, and/or the determination module 230) may analyze the plurality of images. In some embodiments, the processing device 120 may achieve operation 1203 by performing process 300 as described in connection with FIG. 3.

In 1204, the processing device 120 (e.g., the determination module 230) may determine a first value range of a first parameter based on the scan data of the target subject. The processing device 120 may determine values of the first parameter of physical points of the target subject based on-the scan data of the target subject and further determine the first value range of the first parameter based on the values of the first parameter.

In 1205, the processing device 120 (e.g., the determination module 230) may divide a first value range of a first parameter into multiple subranges.

In 1206, for each of the subranges, the processing device 120 (e.g., the determination module 230) may determine a second value range of a second parameter corresponding to the subrange.

In 1207, the processing device 120 (e.g., the generation module 220) may generate a first target image of the target subject based on the scan data and the second value range of the second parameter corresponding to each of the subranges.

In some embodiments, operations 1205, 1206, and 1207 may be performed in a similar manner to operations 610, 620, and 630 as described in connection with FIG. 6, respectively.

In 1208, the processing device 120 (e.g., the obtaining module 210) may obtain basic information of the target subject.

In 1209, the processing device 120 (e.g., the obtaining module 210) may obtain disease risk information of the target subject based on the basic information of the target subject from a disease information database.

In 1210, the processing device 120 (e.g., the generation module 220) may generate a second target image of the target subject based on the disease risk information of the target subject and an initial medical image generated based on the scan data.

In some embodiments, operations 1208, 1209, and 1210 may be performed in a similar manner to operations 910, 920, and 930 as described in connection with FIG. 9, respectively.

The operations of the illustrated processes 300, 600, 900, 1000, and 1200 presented above are intended to be illustrative. In some embodiments, a process may be accomplished with one or more additional operations not described, and/or without one or more of the operations discussed. Additionally, the order in which the operations of a process described above is not intended to be limiting.

Having thus described the basic concepts, it may be rather apparent to those skilled in the art after reading this detailed disclosure that the foregoing detailed disclosure is intended to be presented by way of example only and is not limiting. Various alterations and improvements may occur and are intended to those skilled in the art, though not expressly stated herein. These alterations and improvements are intended to be suggested by this disclosure within the scope of the appended claims.

Moreover, certain terminology has been used to describe embodiments of the present disclosure. For example, the terms "one embodiment," "an embodiment," and/or "some embodiments" may mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "unit," "module," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction execution system, apparatus, or device. Program code embodied on a computer readable signal medium may be transmitted using any appropriate medium, including wireless, wireline, optical fiber cable, RF, or the like, or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB. NET, Python or the like, conventional procedural programming languages, such as the "C" programming language, Visual Basic, Fortran 2103, Perl, COBOL 2102, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments. For example, although the implementation of various components described above may be embodied in a hardware device, it may also be implemented as a software only solution, for example, an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed object matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±1%, ±5%, ±10%, or ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

In closing, it is to be understood that the embodiments of the application disclosed herein are illustrative of the principles of the embodiments of the application. By way of example, but not of limitation, alternative configurations of the embodiments of the application may be utilized in accordance with the appended claims.

## Claims

1. A method for image analysis implemented on a computing device including at least one processor and at least one storage device, the method comprising:
obtaining (310) a plurality of images (410) of a target subject that are captured consecutively over time, the target subject being injected with tracer and including a target body portion;
for each of the plurality of images (410),
generating (320) a sub-image (421, 422, 423) of the target body portion by segmenting the image; and transforming (330) the sub-image (421, 422, 423) to generate a transformed sub-image in a standard space (430, 560) based on a template image of the target body portion, the standard space (430, 560) including a plurality of template images of a plurality of body portions, wherein
the transforming the sub-image (421, 422, 423) includes:
generating a registered sub-image by registering the sub-image (421, 422, 423) with the template image; and
generating the transformed sub-image by adjusting element values in the registered sub-image based on element values in the sub-image (421, 422. 423); and
the standard space (430, 560) is determined by:
for each of a plurality of sample subjects that are in a preset state and injected with tracer, obtaining sample images (511, 512, 513) of the sample subject captured consecutively over time;
for each of the plurality of body portions, generating segmentation images (521, 531, 541, 522, 532, 542, 523, 533, 543) of the body portion by segmenting the body portion from each of the sample images (511, 512, 513) of each sample subject;
for each of the plurality of body portions,
extracting size information and contour information of the body portion from the segmentation images (521, 531, 541, 522, 532, 542, 523, 533, 543) of the body portion;
determining a standard size and a standard contour of the body portion based on the size information and contour information of the body portion; and
generating the template image (551, 552, 553) of the body portion based on the standard size and the standard contour of the body portion; and
generating the standard space (430, 560) by arranging the template images of the plurality of body portions in a preset manner; and
determining (340) an uptake variation curve (441, 442, 443) indicating a change of tracer uptake in the target body portion over the time based on the transformed sub-images corresponding to the plurality of images (410).

2. The method of claim 1, wherein the method further includes:
obtaining (350) a standard uptake variation curve (571, 572, 573) corresponding to the target body portion; and
generating (360) a comparison result between the uptake variation curve (441, 442, 443) and the standard uptake variation curve (571, 572, 573).

3. The method of claim 2, wherein the comparison result includes at least one of:
a comparison graph indicating the uptake variation curve (441, 442, 443) and the standard uptake variation curve (571, 572, 573), or
difference information relating one or more parameters of the uptake variation curve (441, 442, 443) and the standard uptake variation curve (571, 572, 573).

4. The method of claim 1, wherein an average, a maximum value, and/or a minimum value of the element values in the registered sub-image is an average, a maximum value, or a minimum value of the element values in the sub-image (421, 422, 423), respectively.

5. The method of claim 1, wherein an average, a maximum value, and/or a minimum value of the element values, in the transformed sub-image, of each of a plurality of regions of the target body portion is an average, a maximum value or a minimum value of the element values in a corresponding region in the sub- image (421, 422, 423).

6. The method of claim 1, wherein the uptake variation curve (441, 442, 443) is determined by:
determining a change of a pixel count rate of the target body portion over the time based on the transformed sub-images corresponding to the plurality of images (410);
determining a change of the tracer uptake of the target body portion over the time based on the change of the pixel count rate and a preset calibration coefficient determined based on a device that captures the plurality of images (410); and
generating the uptake variation curve (441, 442, 443) corresponding to the target body portion based on the change of the tracer uptake of the target body portion over time.

7. The method of claim 6, wherein the pixel count rate of the target body portion refers to a ratio of a count of target sub-regions of the target body portion that meet a certain condition to a total count of sub-regions of the target body portion.

8. The method of claim 1, wherein the method further includes generating a standard uptake variation curve (571, 572, 573) of each of the plurality of body portions by:
for each of the plurality of sample subjects,
determining a change of a pixel count rate of the body portion of the sample subject over time based on the segmentation images (521, 531, 541, 522, 532, 542, 523, 533, 543) of the body portion of the sample subject; and
determining a change of a tracer uptake of the body portion of the sample subject over time based on a change of the pixel count rate and a preset calibration coefficient; and
generating a standard uptake variation curve (571, 572, 573) corresponding to the body portion based on the change of the tracer uptake of the body portion of each sample subject.

9. The method of claim 1, wherein the method further comprises:
obtaining a plurality of candidate standard spaces corresponding to different types of subjects; and
determining the standard space (430, 560) from the plurality of candidate standard spaces based on a type of the target subject.

10. The method of claim 1, wherein the tracer uptake relates to at least one of a tracer uptake concentration or a tracer standard uptake value (SUV).

11. The method of claim 1, wherein data related to the standard space (430, 560) is stored in a hash table in a storage device, the hash table includes multiple key-value pairs, a key in a key-value pair is used to store an index of a template image of a body portion, and a value in the key-value pair is used to store information of the template image.

12. The method of claim 1, wherein the standard space (430, 560) refers to a space that includes standard sizes and/or contours of the plurality of body portions of the target subject.

13. A system, comprising:
at least one storage device including a set of instructions; and
at least one processor in communication with the at least one storage device, wherein when executing the set of instructions, the at least one processor causes the system to perform operations including:
obtaining (310) a plurality of images (410) of a target subject that are captured consecutively over time, the target subject being injected with tracer and including a target body portion;
for each of the plurality of images (410),
generating (320) a sub-image (421, 422, 423) of the target body portion by segmenting the image; and
transforming (330) the sub-image (421, 422, 423) to generate a transformed sub-image in a standard space (430, 560) based on a template image of the target body portion, the standard space (430, 560) including a plurality of template images of a plurality of body portions, wherein
the transforming the sub-image (421, 422, 423) includes:
generating a registered sub-image by registering the sub-image (421, 422, 423) with the template image; and
generating the transformed sub-image by adjusting element values in the registered sub-image based on element values in the sub-image (421, 422. 423); and
the standard space (430, 560) is determined by:
for each of a plurality of sample subjects that are in a preset state and injected with tracer, obtaining sample images (511, 512, 513) of the sample subject captured consecutively over time;
for each of the plurality of body portions, generating segmentation images (521, 531, 541, 522, 532, 542, 523, 533, 543) of the body portion by segmenting the body portion from each of the sample images (511, 512, 513) of each sample subject;
for each of the plurality of body portions,
extracting size information and contour information of the body portion from the segmentation images (521, 531, 541, 522, 532, 542, 523, 533, 543) of the body portion;
determining a standard size and a standard contour of the body portion based on the size information and contour information of the body portion; and
generating the template image of the body portion based on the standard size and the standard contour of the body portion; and
generating the standard space (430, 560) by arranging the template images of the plurality of body portions in a preset manner; and
determining (340) an uptake variation curve (441, 442, 443) indicating a change of tracer uptake in the target body portion over the time based on the transformed sub-images corresponding to the plurality of images (410).

14. The system of claim 13,
wherein the operations further include generating a standard uptake variation curve (571, 572, 573) of each of the plurality of body portions by:
for each of the plurality of sample subjects,
determining a change of a pixel count rate of the body portion of the sample subject over time based on the segmentation images (521, 531, 541, 522, 532, 542, 523, 533, 543) of the body portion of the sample subject; and
determining a change of a tracer uptake of the body portion of the sample subject over time based on a change of the pixel count rate and a preset calibration coefficient; and
generating a standard uptake variation curve (571, 572, 573) corresponding to the body portion based on the change of the tracer uptake of the body portion of each sample subject.

15. A non-transitory computer readable medium, comprising executable instructions that, when executed by at least one processor, direct the at least one processor to perform a method, the method comprising:
obtaining (310) a plurality of images (410) of a target subject that are captured consecutively over time, the target subject being injected with tracer and including a target body portion;
for each of the plurality of images (410),
generating (320) a sub-image (421, 422, 423) of the target body portion by segmenting the image; and
transforming (330) the sub-image (421, 422, 423) to generate a transformed sub-image in a standard space (430, 560) based on a template image of the target body portion, the standard space (430, 560) including a plurality of template images of a plurality of body portions, wherein
the transforming the sub-image (421, 422, 423) includes:
generating a registered sub-image by registering the sub-image (421, 422, 423) with the template image; and
generating the transformed sub-image by adjusting element values in the registered sub-image based on element values in the sub-image (421, 422. 423); and
the standard space (430, 560) is determined by:
for each of a plurality of sample subjects that are in a preset state and injected with tracer, obtaining sample images (511, 512, 513) of the sample subject captured consecutively over time;
for each of the plurality of body portions, generating segmentation images (521, 531, 541, 522, 532, 542, 523, 533, 543) of the body portion by segmenting the body portion from each of the sample images (511, 512, 513) of each sample subject;
for each of the plurality of body portions,
extracting size information and contour information of the body portion from the segmentation images (521, 531, 541, 522, 532, 542, 523, 533, 543) of the body portion;
determining a standard size and a standard contour of the body portion based on the size information and contour information of the body portion; and
generating the template image of the body portion based on the standard size and the standard contour of the body portion; and
generating the standard space (430, 560) by arranging the template images of the plurality of body portions in a preset manner; and
determining (340) an uptake variation curve (441, 442, 443) indicating a change of tracer uptake in the target body portion over the time based on the transformed sub-images corresponding to the plurality of images **(410).**

## Patentansprüche

1. Verfahren zur Bildanalyse, das auf einer Rechenvorrichtung implementiert ist, die mindestens einen Prozessor und mindestens eine Speichervorrichtung beinhaltet, wobei das Verfahren Folgendes umfasst:
Erhalten (310) einer Vielzahl von Bildern (410) eines Zielsubjekts, die nacheinander über die Zeit aufgenommen werden, wobei dem Zielsubjekt ein Tracer injiziert wird und es einen Zielkörperabschnitt beinhaltet;
für jedes der Vielzahl von Bildern (410),
Erzeugen (320) eines Teilbilds (421, 422, 423) des Zielkörperabschnitts durch Segmentieren des Bilds; und
Transformieren (330) des Teilbilds (421, 422, 423) zum Erzeugen eines transformierten Teilbilds in einem Standardraum (430, 560) basierend auf einem Vorlagenbild des Zielkörperabschnitts, wobei der Standardraum (430, 560) eine Vielzahl von Vorlagenbildern einer Vielzahl von Körperabschnitten beinhaltet, wobei
das Transformieren des Teilbilds (421, 422, 423) Folgendes beinhaltet:
Erzeugen eines registrierten Teilbilds durch Registrieren des Teilbilds (421, 422, 423) bei dem Vorlagenbild; und
Erzeugen eines transformierten Teilbilds durch Anpassen von Elementwerten im registrierten Teilbild basierend auf Elementwerten im Teilbild (421, 422, 423); und
der Standardraum (430, 560) durch Folgendes bestimmt wird:
für jedes einer Vielzahl von Probensubjekten, die sich in einem voreingestellten Zustand befinden und denen Tracer injiziert wurden, Erhalten nacheinander über die Zeit aufgenommener Probenbilder (511, 512, 513) des Probensubjekts;
für jeden der Vielzahl von Körperabschnitten, Erzeugen von Segmentierungsbildern (521, 531, 541, 522, 532, 542, 523, 533, 543) des Körperabschnitts, indem der Körperabschnitt aus jedem der Probenbilder (511, 512, 513) jedes Probensubjekts segmentiert wird;
für jeden der Vielzahl von Körperabschnitten,
Extrahieren von Größeninformationen und Konturinformationen des Körperabschnitts aus den Segmentierungsbildern (521, 531, 541, 522, 532, 542, 523, 533, 543) des Körperabschnitts;
Bestimmen einer Standardgröße und einer Standardkontur des Körperabschnitts basierend auf den Größeninformationen und Konturinformationen des Körperabschnitts; und
Erzeugen des Vorlagenbilds (551, 552, 553) des Körperabschnitts basierend auf der Standardgröße und der Standardkontur des Körperabschnitts; und
Erzeugen des Standardraums (430, 560) durch Anordnen der Vorlagenbilder der Vielzahl von Körperabschnitten in einer vordefinierten Weise; und
Bestimmen (340) einer Aufnahmevariationskurve (441, 442, 443), die eine Veränderung der Tracer-Aufnahme im Zielkörperabschnitt über die Zeit basierend auf transformierten Teilbildern angibt, die der Vielzahl von Bildern (410) entsprechen.

2. Verfahren nach Anspruch 1, wobei das Verfahren weiter Folgendes beinhaltet:
Erhalten (350) einer Standardaufnahmevariationskurve (571, 572, 573), die dem Zielkörperabschnitt entspricht; und
Erzeugen (360) eines Vergleichsergebnisses zwischen der Aufnahmevariationskurve (441, 442, 443) und der Standardaufnahmevariationskurve (571, 572, 573).

3. Verfahren nach Anspruch 2, wobei das Vergleichsergebnis mindestens eines des Folgenden beinhaltet:
einen Vergleichsgraphen, der die Aufnahmevariationskurve (441, 442, 443) und die Standardaufnahmevariationskurve (571, 572, 573) angibt, oder
Differenzinformationen bezüglich eines oder mehrerer Parameter der Aufnahmevariationskurve (441, 442, 443) und der Standardaufnahmevariationskurve (571, 572, 573).

4. Verfahren nach Anspruch 1, wobei ein Mittelwert, ein Maximalwert und/oder ein Minimalwert der Elementwerte im registrierten Teilbild ein Mittelwert, ein Maximalwert beziehungsweise ein Minimalwert der Elementwerte im Teilbild (421, 422, 423) ist.

5. Verfahren nach Anspruch 1, wobei ein Mittelwert, ein Maximalwert und/oder ein Minimalwert der Elementwerte im transformierten Teilbild jedes einer Vielzahl von Bereichen des Zielkörperabschnitts ein Mittelwert, ein Maximalwert oder ein Minimalwert der Elementwerte in einem entsprechenden Bereich im Teilbild (421, 422, 423) ist.

6. Verfahren nach Anspruch 1, wobei die Aufnahmevariationskurve (441, 442, 443) durch Folgendes bestimmt wird:
Bestimmen einer Veränderung einer Pixelzählrate des Zielkörperabschnitts über die Zeit basierend auf den transformierten Teilbildern, die der Vielzahl von Bildern (410) entsprechen;
Bestimmen einer Veränderung der Tracer-Aufnahme des Zielkörperabschnitts über die Zeit basierend auf der Veränderung der Pixelzählrate und einem voreingestellten Kalibrierungskoeffizienten, der basierend auf einer Vorrichtung bestimmt wird, die die Vielzahl von Bildern (410) aufnimmt; und
Erzeugen der Aufnahmevariationskurve (441, 442, 443) entsprechend dem Zielkörperabschnitt, basierend auf der Veränderung der Tracer-Aufnahme des Zielkörperabschnitts über die Zeit.

7. Verfahren nach Anspruch 6, wobei sich die Pixelzählrate des Zielkörperabschnitts auf ein Verhältnis einer Anzahl von Zielteilbereichen des Zielkörperabschnitts, die eine bestimmte Bedingung erfüllen, zu einer Gesamtzahl von Teilbereichen des Zielkörperabschnitts bezieht.

8. Verfahren nach Anspruch 1, wobei das Verfahren weiter Erzeugen einer Standardaufnahmevariationskurve (571, 572, 573) für jeden der Vielzahl von Körperabschnitten beinhaltet durch:
für jedes der Vielzahl von Probensubjekten,
Bestimmen einer Veränderung einer Pixelzählrate des Körperabschnitts des Probensubjekts über die Zeit basierend auf den Segmentierungsbildern (521, 531, 541, 522, 532, 542, 523, 533, 543) des Körperabschnitts des Probensubjekts; und
Bestimmen einer Veränderung einer Tracer-Aufnahme des Körperabschnitts des Probensubjekts über die Zeit basierend auf einer Veränderung der Pixelzählrate und einem voreingestellten Kalibrierungskoeffizienten; und
Erzeugen einer Standardaufnahmevariationskurve (571, 572, 573) entsprechend dem Körperabschnitt, basierend auf der Veränderung der Tracer-Aufnahme des Körperabschnitts jedes Probensubjekts.

9. Verfahren nach Anspruch 1, wobei das Verfahren weiter Folgendes umfasst:
Erhalten einer Vielzahl von Kandidatenstandardräumen, die verschiedenen Subjekttypen entsprechen; und
Bestimmen des Standardraums (430, 560) aus der Vielzahl von Kandidatenstandardräumen basierend auf einem Typ des Zielsubjekts.

10. Verfahren nach Anspruch 1, wobei sich die Tracer-Aufnahme auf mindestens eines einer Tracer-Aufnahmekonzentration oder eines Tracer-Standardaufnahmewerts (SUV) bezieht.

11. Verfahren nach Anspruch 1, wobei Daten, die sich auf den Standardraum (430, 560) beziehen, in einer Hashtabelle in einer Speichervorrichtung gespeichert werden, die Hashtabelle vielfache Schlüssel-Wert-Paare beinhaltet, ein Schlüssel in einem Schlüssel-Wert-Paar zum Speichern eines Index eines Vorlagenbilds eines Körperabschnitts verwendet wird und ein Wert in dem Schlüssel-Wert-Paar zum Speichern von Informationen über das Vorlagenbild verwendet wird.

12. Verfahren nach Anspruch 1, wobei sich der Standardraum (430, 560) auf einen Raum bezieht, der Standardgrößen und/oder -konturen der mehreren Körperabschnitte des Zielsubjekts beinhaltet.

13. System, umfassend:
mindestens eine Speichervorrichtung, die einen Satz von Anweisungen beinhaltet; und
mindestens einen Prozessor, der mit der mindestens einen Speichervorrichtung in Kommunikation steht, wobei, wenn der Satz von Anweisungen ausgeführt wird, der mindestens eine Prozessor das System veranlasst, Operationen durchzuführen, die Folgendes beinhalten:
Erhalten (310) einer Vielzahl von Bildern (410) eines Zielsubjekts, die nacheinander über die Zeit aufgenommen werden, wobei dem Zielsubjekt ein Tracer injiziert wird und es einen Zielkörperabschnitt beinhaltet;
für jedes der Vielzahl von Bildern (410),
Erzeugen (320) eines Teilbilds (421, 422, 423) des Zielkörperabschnitts durch Segmentieren des Bilds; und
Transformieren (330) des Teilbilds (421, 422, 423) zum Erzeugen eines transformierten Teilbilds in einem Standardraum (430, 560) basierend auf einem Vorlagenbild des Zielkörperabschnitts, wobei der Standardraum (430, 560) eine Vielzahl von Vorlagenbildern einer Vielzahl von Körperabschnitten beinhaltet, wobei
das Transformieren des Teilbilds (421, 422, 423) Folgendes beinhaltet:
Erzeugen eines registrierten Teilbilds durch Registrieren des Teilbilds (421, 422, 423) bei dem Vorlagenbild; und
Erzeugen des transformierten Teilbilds durch Anpassen von Elementwerten im registrierten Teilbild basierend auf Elementwerten im Teilbild (421, 422, 423); und
der Standardraum (430, 560) durch Folgendes bestimmt wird:
für jedes einer Vielzahl von Probensubjekten, die sich in einem voreingestellten Zustand befinden und denen Tracer injiziert wurden, Erhalten nacheinander über die Zeit aufgenommener Probenbilder (511, 512, 513) des Probensubjekts;
für jeden der Vielzahl von Körperabschnitten, Erzeugen von Segmentierungsbildern (521, 531, 541, 522, 532, 542, 523, 533, 543) des Körperabschnitts, indem der Körperabschnitt aus jedem der Probenbilder (511, 512, 513) jedes Probensubjekts segmentiert wird;
für jeden der Vielzahl von Körperabschnitten,
Extrahieren von Größeninformationen und Konturinformationen des Körperabschnitts aus den Segmentierungsbildern (521, 531, 541, 522, 532, 542, 523, 533, 543) des Körperabschnitts;
Bestimmen einer Standardgröße und einer Standardkontur des Körperabschnitts basierend auf den Größeninformationen und Konturinformationen des Körperabschnitts; und
Erzeugen des Vorlagenbilds des Körperabschnitts basierend auf der Standardgröße und der Standardkontur des Körperabschnitts; und
Erzeugen des Standardraums (430, 560) durch Anordnen der Vorlagenbilder der Vielzahl von Körperabschnitten in einer vordefinierten Weise; und
Bestimmen (340) einer Aufnahmevariationskurve (441, 442, 443), die eine Veränderung der Tracer-Aufnahme im Zielkörperabschnitt über die Zeit basierend auf transformierten Teilbildern angibt, die der Vielzahl von Bildern (410) entsprechen.

14. System nach Anspruch 13, wobei die Operationen weiter Erzeugen einer Standardaufnahmevariationskurve (571, 572, 573) für jeden der Vielzahl von Körperabschnitten beinhalten durch:
für jedes der Vielzahl von Probensubjekten,
Bestimmen einer Veränderung einer Pixelzählrate des Körperabschnitts des Probensubjekts über die Zeit basierend auf den Segmentierungsbildern (521, 531, 541, 522, 532, 542, 523, 533, 543) des Körperabschnitts des Probensubjekts; und
Bestimmen einer Veränderung einer Tracer-Aufnahme des Körperabschnitts des Probensubjekts über die Zeit basierend auf einer Veränderung der Pixelzählrate und einem voreingestellten Kalibrierungskoeffizienten; und
Erzeugen einer Standardaufnahmevariationskurve (571, 572, 573) entsprechend dem Körperabschnitt, basierend auf der Veränderung der Tracer-Aufnahme des Körperabschnitts jedes Probensubjekts.

15. Nichtflüchtiges computerlesbares Medium, das ausführbare Anweisungen umfasst, die, wenn sie von mindestens einem Prozessor ausgeführt werden, den mindestens einen Prozessor anweisen, ein Verfahren durchzuführen, wobei das Verfahren umfasst:
Erhalten (310) einer Vielzahl von Bildern (410) eines Zielsubjekts, die nacheinander über die Zeit aufgenommen werden, wobei dem Zielsubjekt ein Tracer injiziert wird und es einen Zielkörperabschnitt beinhaltet;
für jedes der Vielzahl von Bildern (410),
Erzeugen (320) eines Teilbilds (421, 422, 423) des Zielkörperabschnitts durch Segmentieren des Bilds; und
Transformieren (330) des Teilbilds (421, 422, 423) zum Erzeugen eines transformierten Teilbilds in einem Standardraum (430, 560) basierend auf einem Vorlagenbild des Zielkörperabschnitts, wobei der Standardraum (430, 560) eine Vielzahl von Vorlagenbildern einer Vielzahl von Körperabschnitten beinhaltet, wobei
das Transformieren des Teilbilds (421, 422, 423) Folgendes beinhaltet:
Erzeugen eines registrierten Teilbilds durch Registrieren des Teilbilds (421, 422, 423) bei dem Vorlagenbild; und
Erzeugen des transformierten Teilbilds durch Anpassen von Elementwerten im registrierten Teilbild basierend auf Elementwerten im Teilbild (421, 422, 423); und
der Standardraum (430, 560) durch Folgendes bestimmt wird:
für jedes einer Vielzahl von Probensubjekten, die sich in einem voreingestellten Zustand befinden und denen Tracer injiziert wurden, Erhalten nacheinander über die Zeit aufgenommener Probenbilder (511, 512, 513) des Probensubjekts;
für jeden der Vielzahl von Körperabschnitten, Erzeugen von Segmentierungsbildern (521, 531, 541, 522, 532, 542, 523, 533, 543) des Körperabschnitts, indem der Körperabschnitt aus jedem der Probenbilder (511, 512, 513) jedes Probensubjekts segmentiert wird;
für jeden der Vielzahl von Körperabschnitten,
Extrahieren von Größeninformationen und Konturinformationen des Körperabschnitts aus den Segmentierungsbildern (521, 531, 541, 522, 532, 542, 523, 533, 543) des Körperabschnitts;
Bestimmen einer Standardgröße und einer Standardkontur des Körperabschnitts basierend auf den Größeninformationen und Konturinformationen des Körperabschnitts; und
Erzeugen des Vorlagenbilds des Körperabschnitts basierend auf der Standardgröße und der Standardkontur des Körperabschnitts; und
Erzeugen des Standardraums (430, 560) durch Anordnen der Vorlagenbilder der Vielzahl von Körperabschnitten in einer vordefinierten Weise; und
Bestimmen (340) einer Aufnahmevariationskurve (441, 442, 443), die eine Veränderung der Tracer-Aufnahme im Zielkörperabschnitt über die Zeit basierend auf transformierten Teilbildern angibt, die der Vielzahl von Bildern (410) entsprechen.

## Revendications

1. Procédé d'analyse d'image mis en œuvre sur un dispositif informatique incluant au moins un processeur et au moins un dispositif de stockage, le procédé comprenant :
l'obtention (310) d'une pluralité d'images (410) d'un sujet cible qui sont capturées consécutivement au fil du temps, le sujet cible étant injecté avec un traceur et incluant une partie de corps cible ;
pour chaque image de la pluralité d'images,
la génération (320) d'une sous-image (421, 422, 423) de la partie de corps cible en segmentant l'image ; et
la transformation (330) de la sous-image (421, 422, 423) pour générer une sous-image transformée dans un espace standard (430, 560) sur la base d'une image modèle de la partie de corps cible, l'espace standard (430, 560) incluant une pluralité d'images modèles d'une pluralité de parties de corps, dans lequel
la transformation de la sous-image (421, 422, 423) inclut :
la génération d'une sous-image enregistrée en enregistrant la sous-image (421, 422, 423) avec l'image modèle ; et
la génération de la sous-image transformée en ajustant des valeurs d'élément dans la sous-image enregistrée sur la base de valeurs d'élément dans la sous-image (421, 422, 423) ; et
l'espace standard (430, 560) est déterminé en :
pour chaque sujet d'échantillon d'une pluralité de sujets d'échantillon qui sont dans un état prédéfini et injectés avec un traceur, obtenant des images d'échantillon (511, 512, 513) du sujet d'échantillon capturées consécutivement au fil du temps ;
pour chaque partie de corps de la pluralité de parties de corps, générant des images de segmentation (521, 531, 541, 522, 532, 542, 523, 533, 543) de la partie de corps en segmentant la partie de corps à partir de chacune des images d'échantillon (511, 512, 513) de chaque sujet échantillon ;
pour chaque partie de corps de la pluralité de parties de corps,
extrayant des informations de taille et des informations de contour de la partie de corps à partir des images de segmentation (521, 531, 541, 522, 532, 542, 523, 533, 543) de la partie de corps ;
déterminant une taille standard et un contour standard de la partie de corps sur la base des informations de taille et des informations de contour de la partie de corps ; et
générant l'image modèle (551, 552, 553) de la partie de corps sur la base de la taille standard et du contour standard de la partie de corps ; et
générant l'espace standard (430, 560) en agençant les images modèles de la pluralité de parties de corps de manière prédéfinie ; et
déterminant (340) une courbe de variation d'absorption (441, 442, 443) indiquant un changement d'absorption de traceur dans la partie de corps cible au **fil du** temps sur la base des sous-images transformées correspondant à la pluralité d'images (410).

2. Procédé selon la revendication 1, dans lequel le procédé inclut en outre :
l'obtention (350) d'une courbe de variation d'absorption standard (571, 572, 573) correspondant à la partie de corps cible ; et
la génération (360) d'un résultat de comparaison entre la courbe de variation d'absorption (441, 442, 443) et la courbe de variation d'absorption standard (571, 572, 573).

3. Procédé selon la revendication 2, dans lequel le résultat de comparaison inclut au moins un :
d'un graphique de comparaison indiquant la courbe de variation d'absorption (441, 442, 443) et la courbe de variation d'absorption standard (571, 572, 573), ou
d'informations de différence se rapportant à un ou plusieurs paramètres de la courbe de variation d'absorption (441, 442, 443) et la courbe de variation d'absorption standard (571, 572, 573).

4. Procédé selon la revendication 1, dans lequel une moyenne, une valeur maximale et/ou une valeur minimale des valeurs d'élément dans la sous-image enregistrée est une moyenne, une valeur maximale ou une valeur minimale des valeurs d'élément dans la sous-image (421, 422, 423), respectivement.

5. Procédé selon la revendication 1, dans lequel une moyenne, une valeur maximale et/ou une valeur minimale des valeurs d'élément, dans la sous-image transformée, de chaque région d'une pluralité de régions de la partie de corps cible est une moyenne, une valeur maximale ou une valeur minimale des valeurs d'élément dans une région correspondante de la sous-image (421, 422, 423).

6. Procédé selon la revendication 1, dans lequel la courbe de variation d'absorption (441, 442, 443) est déterminée en :
déterminant un changement d'un taux de comptage de pixels de la partie de corps cible au fil du temps sur la base des sous-images transformées correspondant à la pluralité d'images (410) ;
déterminant un changement de l'absorption de traceur par la partie de corps cible au fil du temps sur la base du changement du taux de comptage de pixels et d'un coefficient d'étalonnage prédéfini déterminé sur la base d'un dispositif qui capture la pluralité d'images (410) ; et
générant la courbe de variation d'absorption (441, 442, 443) correspondant à la partie de corps cible sur la base de la variation de l'absorption de traceur de la partie de corps cible au fil du temps.

7. Procédé selon la revendication 6, dans lequel le taux de comptage de pixels de la partie de corps cible fait référence à un rapport entre le nombre de sous-régions cibles de la partie de corps cible qui répondent à une certaine condition et le nombre total de sous-régions de la partie de corps cible.

8. Procédé selon la revendication 1, dans lequel le procédé inclut en outre la génération d'une courbe de variation d'absorption standard (571, 572, 573) de chaque partie de corps de la pluralité de parties de corps en :
pour chaque sujet d'échantillon de la pluralité de sujets d'échantillon,
déterminant un changement du taux de comptage de pixels de la partie de corps du sujet d'échantillon au fil du temps, sur la base des images de segmentation (521, 531, 541, 522, 532, 542, 523, 533, 543) de la partie de corps du sujet d'échantillon ; et
déterminant un changement de l'absorption de traceur de la partie de corps du sujet d'échantillon au fil du temps, sur la base d'un changement du taux de comptage de pixels et d'un coefficient d'étalonnage prédéfini ; et
générant une courbe de variation d'absorption standard (571, 572, 573) correspondant à la partie de corps sur la base du changement de l'absorption de traceur de la partie de corps de chaque sujet d'échantillon.

9. Procédé selon la revendication 1, dans lequel le procédé inclut en outre :
l'obtention d'une pluralité d'espaces standards candidats correspondant à différents types de sujets ; et
la détermination de l'espace standard (430, 560) à partir de la pluralité d'espaces standards candidats sur la base d'un type du sujet cible.

10. Procédé selon la revendication 1, dans lequel l'absorption de traceur se rapporte à au moins une concentration d'absorption de traceur ou à une valeur d'absorption standard de traceur (SUV).

11. Procédé selon la revendication 1, dans lequel des données se rapportant à l'espace standard (430, 560) sont stockées dans une table de hachage dans un dispositif de stockage, la table de hachage inclut de multiples paires clé-valeur, une clé dans une paire clé-valeur est utilisée pour stocker un index d'une image modèle d'une partie de corps, et une valeur dans la paire clé-valeur est utilisée pour stocker des informations de l'image modèle.

12. Procédé selon la revendication 1, dans lequel l'espace standard (430, 560) se réfère à un espace qui inclut des tailles standards et/ou des contours standards de la pluralité de parties de corps du sujet cible.

13. Système, comprenant :
au moins un dispositif de stockage incluant un ensemble d'instructions ; et
au moins un processeur en communication avec le au moins un dispositif de stockage, dans lequel, lors de l'exécution de l'ensemble d'instructions, le au moins un processeur amène le système à effectuer des opérations incluant :
l'obtention (310) d'une pluralité d'images (410) d'un sujet cible qui sont capturées consécutivement au fil du temps, le sujet cible étant injecté avec un traceur et incluant une partie de corps cible ;
pour chaque image de la pluralité d'images,
la génération (320) d'une sous-image (421, 422, 423) de la partie de corps cible en segmentant l'image ; et
la transformation (330) de la sous-image (421, 422, 423) pour générer une sous-image transformée dans un espace standard (430, 560) sur la base d'une image modèle de la partie de corps cible, l'espace standard (430, 560) incluant une pluralité d'images modèles d'une pluralité de parties de corps, dans lequel
la transformation de la sous-image (421, 422, 423) inclut :
la génération d'une sous-image enregistrée en enregistrant la sous-image (421, 422, 423) avec l'image modèle ; et
la génération de la sous-image transformée en ajustant des valeurs d'élément dans la sous-image enregistrée sur la base de valeurs d'élément dans la sous-image (421, 422, 423) ; et
l'espace standard (430, 560) est déterminé en :
pour chaque sujet d'échantillon d'une pluralité de sujets d'échantillon qui sont dans un état prédéfini et injectés avec un traceur, obtenant des images d'échantillon (511, 512, 513) du sujet d'échantillon capturées consécutivement au fil du temps ;
pour chaque partie de corps de la pluralité de parties de corps, générant des images de segmentation (521, 531, 541, 522, 532, 542, 523, 533, 543) de la partie de corps en segmentant la partie de corps à partir de chacune des images d'échantillon (511, 512, 513) de chaque sujet échantillon ;
pour chaque partie de corps de la pluralité de parties de corps,
extrayant des informations de taille et des informations de contour de la partie de corps à partir des images de segmentation (521, 531, 541, 522, 532, 542, 523, 533, 543) de la partie de corps ;
déterminant une taille standard et un contour standard de la partie de corps sur la base des informations de taille et des informations de contour de la partie de corps ; et
générant l'image modèle de la partie de corps sur la base de la taille standard et du contour standard de la partie de corps ; et
générant l'espace standard (430, 560) en agençant les images modèles de la pluralité de parties de corps de manière prédéfinie ; et
déterminant (340) une courbe de variation d'absorption (441, 442, 443) indiquant un changement d'absorption de traceur dans la partie de corps cible au fil du temps sur la base des sous-images transformées correspondant à la pluralité d'images (410).

14. Système selon la revendication 13, dans lequel les opérations incluent en outre la génération d'une courbe de variation d'absorption standard (571, 572, 573) de chaque partie de corps des parties de corps en :
pour chaque sujet d'échantillon de la pluralité de sujets d'échantillon,
déterminant un changement du taux de comptage de pixels de la partie de corps du sujet d'échantillon au fil du temps, sur la base des images de segmentation (521, 531, 541, 522, 532, 542, 523, 533, 543) de la partie de corps du sujet d'échantillon ; et
déterminant un changement de l'absorption de traceur de la partie de corps du sujet d'échantillon au fil du temps sur la base d'un changement du taux de comptage de pixels et d'un coefficient d'étalonnage prédéfini ; et
générant une courbe de variation d'absorption standard (571, 572, 573) correspondant à la partie de corps sur la base du changement de l'absorption de traceur de la partie de corps de chaque sujet d'échantillon.

15. Support non transitoire lisible par ordinateur, comprenant des instructions exécutables qui, lorsqu'elles sont exécutées par au moins un processeur, ordonnent à le au moins un processeur de réaliser un procédé, le procédé comprenant :
l'obtention (310) d'une pluralité d'images (410) d'un sujet cible qui sont capturées consécutivement au fil du temps, le sujet cible étant injecté avec un traceur et incluant une partie de corps cible ;
pour chaque image de la pluralité d'images,
la génération (320) d'une sous-image (421, 422, 423) de la partie de corps cible en segmentant l'image ; et
la transformation (330) de la sous-image (421, 422, 423) pour générer une sous-image transformée dans un espace standard (430, 560) sur la base d'une image modèle de la partie de corps cible, l'espace standard (430, 560) incluant une pluralité d'images modèles d'une pluralité de parties de corps, dans lequel
la transformation de la sous-image (421, 422, 423) inclut :
la génération d'une sous-image enregistrée en enregistrant la sous-image (421, 422, 423) avec l'image modèle ; et
la génération de la sous-image transformée en ajustant des valeurs d'élément dans la sous-image enregistrée sur la base de valeurs d'élément dans la sous-image (421, 422, 423) ; et
l'espace standard (430, 560) est déterminé en :
pour chaque sujet d'échantillon d'une pluralité de sujets d'échantillon qui sont dans un état prédéfini et injectés avec un traceur, obtenant des images d'échantillon (511, 512, 513) du sujet d'échantillon capturées consécutivement au fil du temps ;
pour chaque partie de corps de la pluralité de parties de corps, générant des images de segmentation (521, 531, 541, 522, 532, 542, 523, 533, 543) de la partie de corps en segmentant la partie de corps à partir de chacune des images d'échantillon (511, 512, 513) de chaque sujet échantillon ;
pour chaque partie de corps de la pluralité de parties de corps,
extrayant des informations de taille et des informations de contour de la partie de corps à partir des images de segmentation (521, 531, 541, 522, 532, 542, 523, 533, 543) de la partie de corps ;
déterminant une taille standard et un contour standard de la partie de corps sur la base des informations de taille et des informations de contour de la partie de corps ; et
générant l'image modèle de la partie de corps sur la base de la taille standard et du contour standard de la partie de corps ; et
générant l'espace standard (430, 560) en agençant les images modèles de la pluralité de parties de corps de manière prédéfinie ; et
déterminant (340) une courbe de variation d'absorption (441, 442, 443) indiquant un changement d'absorption de traceur dans la partie de corps cible au **fil du** temps sur la base des sous-images transformées correspondant à la pluralité d'images (410).
